(19) Europäisches Patentamt
European Patent Office
Office européen des brevets

(11) **EP 3 076 892 B1**

(12) **EUROPEAN PATENT SPECIFICATION**

(45) Date of publication and mention
of the grant of the patent:
**16.10.2019 Bulletin 2019/42**

(21) Application number: **14863296.1**

(22) Date of filing: **20.11.2014**

(51) Int Cl.:
*A61B 34/20* (2016.01)   *A61B 90/00* (2016.01)
*G01S 5/16* (2006.01)   *G01B 11/14* (2006.01)

(86) International application number:
**PCT/IL2014/051011**

(87) International publication number:
**WO 2015/075720 (28.05.2015 Gazette 2015/21)**

(54) **A MEDICAL OPTICAL TRACKING SYSTEM**

MEDIZINISCHES OPTISCHES VERFOLGUNGSSYSTEM

SYSTÈME MÉDICAL DE SUIVI OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2013 IL 22952713**
**14.08.2014 US 201462037132 P**

(43) Date of publication of application:
**12.10.2016 Bulletin 2016/41**

(73) Proprietor: **Elbit Systems Ltd.**
**3100401 Haifa (IS)**

(72) Inventors:
• **BEN-YISHAI, Rani**
**64284 Tel Aviv (IL)**
• **CHARNY, Adi**
**34765 Hod Hasharon (IL)**
• **YAHAV, Dror**
**Kfar-Saba 4465204 (IL)**
• **ZOMMER, Shahaf**
**Haifa 3269308 (IL)**
• **EFRAT, Ilan**
**34367 Haifa (IL)**

(74) Representative: **Schuhmann, Albrecht**
**c/o Merten Patentmanagement**
**Alexanderstrasse 1**
**90547 Stein (DE)**

(56) References cited:
WO-A1-2009/040792   WO-A1-2012/001550
US-A1- 2004 138 556   US-A1- 2011 079 703
US-A1- 2012 236 993

## Description

### FIELD OF THE DISCLOSED TECHNIQUE

The disclosed technique relates to tracking systems, in general, and to optical tracking system for determining the position and orientation of a moving object, in particular.

### BACKGROUND OF THE DISCLOSED TECHNIQUE

Optical tracking systems for tracking the position and orientation of a moving object in a reference coordinate system are known in the art. These tracking devices employ optical detectors (e.g., Charge Coupled Devices) for gathering information about the position and/or orientation of a moving object. One configuration for such an optical tracking device is fixing one or several optical detectors on the moving object and fixing a set of light sources (e.g., Light Emitting Diodes) at a known position in the coordinate system. Another configuration for such an optical tracking device is fixing a set of light sources on the moving object and fixing one or several optical detectors at a known position in the reference coordinate system. Yet another configuration is combining the former configurations and fixing both detectors and light emitters on the moving object and at a known position in the reference coordinate system. Optical tracking systems enable automatic decision making based of the determined position and/or orientation. For example, a pilot may aim at a target by moving only her head toward the target (i.e., the pilot does not have to move the aircraft toward the target). The optical tracking system determines the orientation (i.e., elevation, azimuth and roll) of the helmet, worn by the pilot, in the aircraft coordinate system. As a further example, the optical tracking system may track the movements of a user of a virtual reality system (e.g., a game, a simulator) determining the position of the user.

However, an optical detector placed on the moving object can detect the light emitters in the reference coordinate system only as long as the light emitters are within the Field Of View (FOV) of the detector. Therefore, the FOV of the optical tracking system (i.e., the range of positions in which the optical tracking system tracks the moving object) is limited by the FOV of the optical detector. Similarly, the fixed light detector can track the moving object as long as the light emitters attached to the moving object are within the FOV of the fixed light detector. Thus, the intersection of the FOV of the moving light detector, with the FOV of the fixed light detector, defines the tracking space of the tracking system.

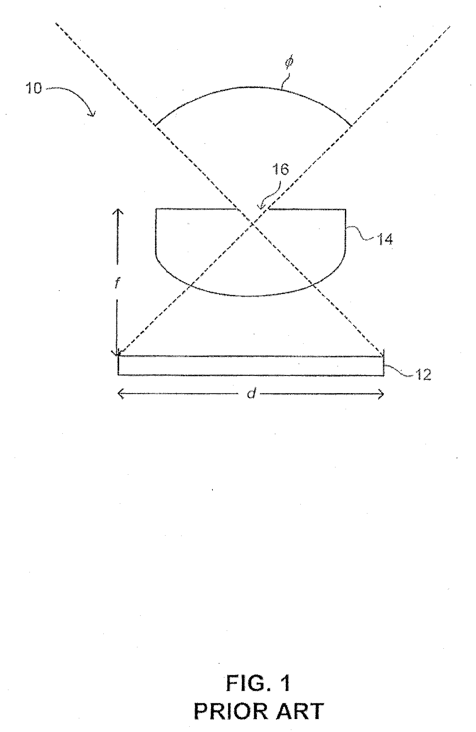

Reference is now made to Figure 1, which is a schematic illustration of an optical detector, generally referenced 10, which is known in the art. Optical detector 10 includes an optical sensor 12 optically coupled with a lens 14. Lens 14 includes an entrance pupil 16. The FOV $\phi$ of optical detector 10 is inversely proportional to the ratio between the focal length $f$ of lens 14 and the size $d$ of optical sensor 12. Furthermore, the accuracy of optical detector 10 is proportional to the angular resolution thereof. Therefore, when the size of sensor 12 (e.g., number of pixels) is fixed, increasing the focal length of lens 14, increases the resolution but decreases the FOV of optical detector 10.

U.S. Patent No. 3,678,283 issued to LaBaw, and entitled "Radiation Sensitive Optical Tracker", is directed to a system for determining the sight line of a pilot with respect to a point in a cockpit. The optical tracker includes: two detector assemblies and three light emitters. The first detector assembly is mounted on the helmet of the pilot. The first light emitter is mounted on the helmet of the pilot. The second detector assembly is mounted on the cockpit, at the point. The second and third light emitters are mounted on the cockpit, equally spaced on either side of the bore sight line in front of the pilot.

The detector assemblies include lateral photo detectors able to detect the lateral position of the light spot. The light emitters illuminate at a light frequency corresponding to the maximum sensitivity range of the detectors. The two light emitters mounted on the cockpit illuminate the detector mounted on the helmet. The illuminator mounted on the helmet illuminates the detector mounted on the cockpit. The determination of the azimuth and elevation angles, of the line of sight of the pilot, is irrespective of the helmet position within the cockpit. The amount of roll of the head of the pilot is computed by the output of the helmet mounted detector, which detects the two cockpit mounted light emitters.

U.S. Patent No. 5,767,524 issued to Barbier et al., and entitled "Optical Device for Determining the Orientation of a Solid Body", is directed to a system for determining the orientation of a first solid body with respect to a second solid body. The orientation determination system includes: three sets of optical source/detector. Each optical source/detector set includes an optical source and an optical radiation detector. At least one source/detector set is mounted on the first solid body. At least one source/detector set is mounted on the second solid body. On at least one of the solid bodies there are mounted two source/detector sets.

The orientation system determines in the first referential system, of the first solid body, two straight lines corresponding to the light radiation coming from the second referential system. The orientation system determines in the second referential system, of the second solid body, two straight lines corresponding to the light radiation coming from the first referential system. The knowledge of the orientation of at least two distinct straight lines in each of the referential systems gives, by computation of the rotation matrix, the three parameters of orientation of the first solid body with respect to the referential system of the second solid body.

P.C.T Application Publication WO 2009/040792 to Yahav et al, directs to an optical tracking system for determining the position and orientation of a moving object in a reference coordinate system. The system includes light emitters and optical detectors. A processor determines the position and orientation of the moving object according to the light detected by the optical detectors. One of the optical detectors and at least one of the light emitters are situated at a fixed position in the reference coordinate system. The other of the optical detectors and at least one other light emitter are attached to the moving object. Either one of the optical detectors may be a wide field of view optical detector.

U.S. Patent Application Publication U.S. 2004/0138665 to Cosman, directs to a system for optically tracking an instrument relative to the anatomy of a patient in a clinical field of view. The system includes two spatially separated cameras, an instrument, data storage and a computer. The two cameras are capable of viewing the clinical field of view to provide camera data in a first coordinate system defined by the camera system. The instrument includes an optically detectable object that is detectable by the camera system to provide instrument data representative of the position of the instrument in the first coordinate system. The data storage includes image data representative of the anatomy of the patient received from an imaging machine. The computer receives the camera data, the instrument data, and the image data and transforms the image data, the camera data, and the instrument data into a second coordinate system, thereby generating tracking data representative of the position of the instrument in relation to the anatomy of the patient.

U.S. Patent Application Publication U.S. 2011/0079703 to Gunning et al, directs to An object tracking system, which includes at least one point source emitter arranged to output respective optical emissions and at least one angle of arrival sensor. Each of the sensors includes a focal plane array (FPA), and at least one optical element transforming the optical emissions into one or more linear patterns on the FPA. The Field Of View (FOV) of the sensors encompasses at least one of the optical emissions such that the sensor detects at least one of the linear patterns. A processer coupled with the sensors, establishes the orientation and position of the point source emitters within each sensor's FOV using the detected linear patterns.

## SUMMARY OF THE PRESENT DISCLOSED TECHNIQUE

It is an object of the disclosed technique to provide a novel system determining the position and orientation of a target object in a reference coordinate system.

In accordance with one aspect of the disclosed technique, there is thus provided a medical WFOV optical tracking system for determining the position and orientation of a target object in a reference coordinate. The system includes at least one light emitter attached to the target object, at least one other light emitter attached to a reference location, a Wide Field Of View optical detector, another optical detector and a processor. The processor is wirelessly coupled with at least one of the at least one Wide Field of View optical detector and the optical detector and further coupled the other ones of the at least one Wide Field of View optical detector and the at least one other optical detector. The reference location is associated with the reference coordinate system. The Wide Field Of View optical detector acquires at least one image of at least one light emitter within the field of view thereof. The Wide Field Of View optical detector includes an optical sensor, for sensing light received from at least one of the at least one light emitter within the field of view of the Wide Field Of View optical detector and at least two optical receptors, optically coupled with the optical sensor. Each of the optical receptors includes an entrance pupil. The optical receptors are spatially spaced apart from each other. Each of the optical receptors projects a different angular section of an observed scene on the optical sensor. The other optical detector acquires at least one image of at least one light emitter within the field of view thereof. The processor determines the position and orientation of each of each target object in the reference coordinate system, according to representations of the at least one light emitter attached to the target object and the at least one other light emitter attached to the reference location. The target object and the reference location are one of respective elements in a tuple including two elements from a group consisting of a display, a patient body location, a medical tool, a physician body location and a fixed position. Each WFOV optical detector and another optical detector is attached to a respective one of the elements. One of the elements is designated as a reference location and the remaining ones are designated as target objects. The total number of light emitters is at least three.

In accordance with another aspect of the disclosed technique, there is thus provided a medical WFOV optical tracking system for determining the position and orientation of a target object in a reference coordinate. The system includes at least one light emitter attached to the target object, at least two light emitters attached to a head mounted display, a Wide Field Of View optical detector, an optical detector and a processor. The processor is coupled with the Wide Field of View optical detector, with the optical detector and with the head mounted display. The target object is of one of at least one of a patient and a medical tool. The head mounted display is located on the head of the physician and is associated with a reference coordinate system. The Wide Field Of View optical detector is attached to the target object and acquires at least one image of each at least two light emitters attached to the head mounted display within the field of view thereof. The Wide Field Of View optical detector includes an optical sensor, for sensing light received from at least one of the at least two light emitters attached to the head mounted display. Each of the optical receptors includes an entrance pupil. The optical receptors are spatially spaced apart from each other. Each of the optical receptors projects a different angular section of an observed scene on the optical sensor. The optical detector is attached to the head mounted display and acquires at least one image of each of the at least one light emitter attached to the target object within the field of view thereof. The processor determines the position and orientation of each of each target object in the reference coordinate system, according to representations of the at least one light emitter attached to the target object and the at least one other light emitter attached to the reference location. The head mounted display displays a least one rendered model of the patient and a representation the medical tool, to the physician, at an orientation corresponding to the determined orientation of the at least one selected target object.

**BRIEF DESCRIPTION OF THE DRAWINGS**

The disclosed technique will be understood and appreciated more fully from the following detailed description taken in conjunction with the drawings in which figures 1-8 relate to background art and in which figures 10-12 show the invention.

Figure 1 is a schematic illustration of an optical detector, which is known in the art;
Figures 2A and 2B are schematic illustrations of a WFOV optical detector assembly, constructed and operative in accordance with an embodiment of the disclosed technique;
Figures 3A and 3B are schematic illustrations of WFOV optical detector assembly, constructed and operative in accordance with another embodiment of the disclosed technique;
Figure 4 is a schematic illustration of an optical tracking system, for determining the pose (i.e., position and orientation) of a moving object in a reference coordinate system in accordance with a further embodiment of the disclosed technique;
Figures 5A, 5B, 5C and 5D are schematic illustrations of images of a single light emitter acquired by a WFOV optical detector which includes only two adjacent optical receptors;
Figure 6 is an example for determining the horizontal orientation of a moving object without determining the position thereof in accordance with another embodiment of the disclosed technique;
Figure 7 is a schematic illustration of an optical tracking system, constructed and operative in accordance with a further embodiment of the disclosed technique;
Figure 8 is a schematic illustration of a two-dimensional example for determining the orientation of a moving object without determining the position thereof in accordance with another embodiment of the disclosed technique;
Figure 9 is a schematic illustration of an exemplary medical WFOV medical tracking system, in accordance with a further embodiment of the disclosed technique;
Figure 10 is a schematic illustration of an exemplary medical WFOV medical tracking system, in accordance with the invention;
Figure 11 is a schematic illustration of an exemplary medical WFOV medical tracking system, in accordance with the invention;
Figure 12 is a schematic illustration of an exemplary medical WFOV medical tracking system, in accordance with the invention;
Figure 13 is a schematic illustration of an exemplary medical WFOV medical tracking system, in accordance with a further embodiment of the disclosed technique;
Figure 14 is a schematic illustration of an exemplary medical WFOV medical tracking system, in accordance with another embodiment of the disclosed technique;
Figure 15, is a schematic illustration of an optical detector assembly and the operation thereof, in accordance with a further embodiment of the disclosed technique;
Figures 16A-16E are schematic illustration of an optical detector assembly and the operation thereof, in accordance with another embodiment of the disclosed technique;
Figure 17 is a diagram illustrating an aspect of a system according to embodiments of the disclosed technique;
Figure 18 is a diagram illustrating another aspect of a system according to embodiments of the disclosed technique;
Figure 19 is a diagram illustrating non-limiting exemplary applications of the system according to embodiments of the disclosed technique;
Figure 20 is a diagram illustrating another non-limiting exemplary application of the system according to embodiments of the disclosed technique; and
Figures 21A and 21B are diagrams illustrating further non-limiting exemplary applications of the system according to embodiments of the disclosed technique.

**DETAILED DESCRIPTION OF THE EMBODIMENTS**

The disclosed technique overcomes the disadvantages of the prior art by providing an optical tracking system

for determining the pose of a moving object including a moving optical detector and a reference optical detector. The term "pose" relates hereinafter to the position (i.e., the x, y and z coordinates) and the orientation (i.e., azimuth elevation and roll angles). According to one embodiment of the disclosed technique, the moving optical detector exhibits a novel configuration, for increasing the FOV thereof, without increasing the size of the optical sensor or decreasing the focal length of the optical receptor (i.e., which decreases the accuracy of the tracking system). According to another embodiment of the disclosed technique, the spatial setup of the light emitters and the detectors enables the optical tracking system to determine the orientation of a moving object (e.g., a helmet, a stylus, a medical needle, an ultrasound imager), in a reference coordinate system (e.g., the coordinate system of an aircraft), without determining the position of the moving object. According to a further embodiment of the disclosed technique, a reflective surface replaces the reference detector, and also enables the optical tracking system to determine the orientation of a moving object, in a reference coordinate system, without determining the position of the object.

As mentioned above an optical detector, placed on a moving object, can detect light emitters that are situated within the FOV of that optical detector. Therefore, increasing the FOV of the optical detector increases the tracking range of the tracking system. In order to increase the FOV of the optical detector a plurality of optical receptors (e.g., lenses or pinholes or both) are placed over an optical sensor. Additionally, the optical axes of the optical receptors may be unparallel with respect to each other. Thus, the field of view of the detector is increased (i.e., relative to the FOV of a single optical receptor). Furthermore, the focal length of each optical receptor may be different. It is noted that the WFOV optical detector according to the disclosed technique, resolves objects in the WFOV thereof, when the angular span of these objects is substantially small (i.e., point like objects), such that the images of the object, formed on the optical sensor by the various lenses, do not overlap with each other.

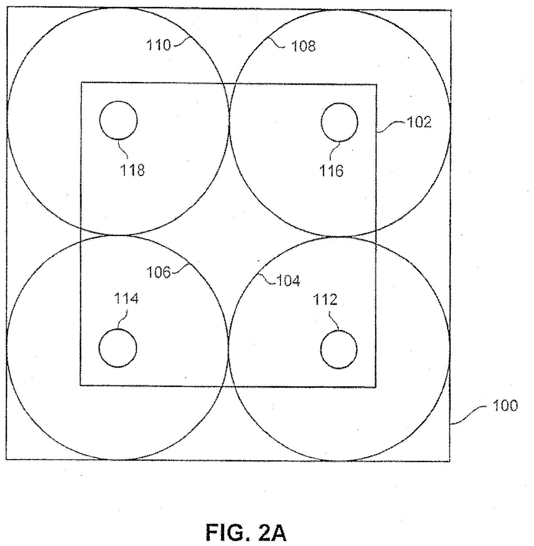

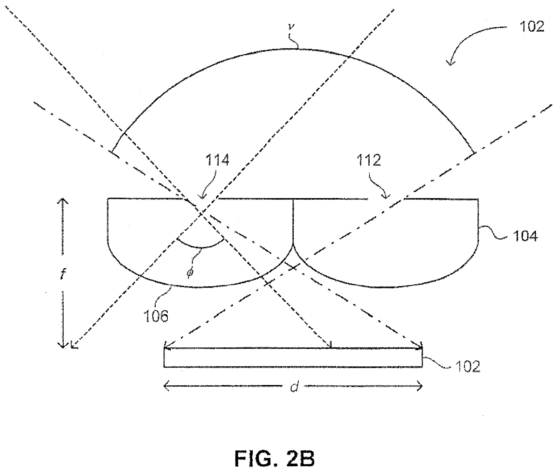

Reference is now made to Figures 2A and 2B, which are schematic illustrations of a WFOV optical detector assembly, generally referenced 100, constructed and operative in accordance with an embodiment of the disclosed technique. Figure 2B is a side view of optical detector assembly 100. Optical detector assembly 100 includes an optical sensor 102 and optical receptors 104, 106, 108 and 110. Optical receptors 104, 106, 108 and 110 are spaced apart from each other. Each one of optical receptors 104, 106, 108 and 110 includes an entrance pupil. Optical receptor 104 includes an entrance pupil 112, optical receptor 106 includes an entrance pupil 114, optical receptor 108 includes an entrance pupil 116 and optical receptor 110 includes an entrance pupil 118. Optical receptors 104, 106, 108 and 110 may be optical lenses. Alternatively, Optical receptors 104, 106, 108 and 110 may be pinholes.

Optical receptors 104, 106, 108 and 110 are optically coupled with optical sensor 102. Optical sensor 102 is, for example, a CCD detector, a Complementary Metal Oxide Semiconductor (CMOS) sensor, a Position Sensitive Device (PSD) or a lateral photo-detector. Optical receptors 104, 106, 108 and 110 are arranged such that each element projects different angular sections of the observed scene (not shown) on the same area of optical sensor 102. The FOV $v$ (Figure 2B), of optical detector assembly 100 is greater than FOV $\phi$ (Figure 2B) of a single optical receptor such as optical receptor 106. Thus, the FOV of optical detector assembly 100 is increased (i.e., relative to the FOV of a single element) without increasing the size $d$ (Figure 2B) of optical detector 100 or decreasing the focal length $f$ (Figure 2B) of optical detector assembly 100.

To increase the resolution at the center of the FOV of the optical detector, an additional optical receptor, with a larger focal length, is placed above the optical receptors. Furthermore, to increase the FOV of the optical detector the bottom optical receptors are tilted, relative to one another, such that the optical axes thereof are unparallel.

Optical detector 100 exhibits a unique response to the direction of light incident thereupon. The position of the light incident on optical sensor 102 is related to the direction from which light enters each of entrance pupils 112, 114, 116 and 118. The unique response of the optical detector to the direction of light incident thereupon is referred to herein as the "directional response". For example, when the optical sensor 102 is a CCD sensor, each pixel in the CCD is associated with an angular step. When the optical sensor is a lateral photo-detector, the current differences at the terminals of the detector are related to the angle of light incident on the lateral photo-detector.

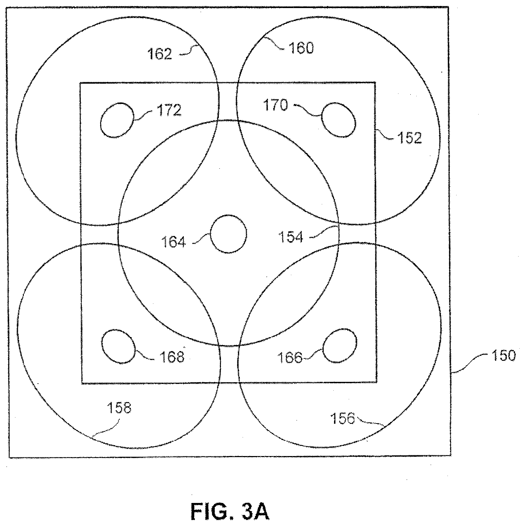

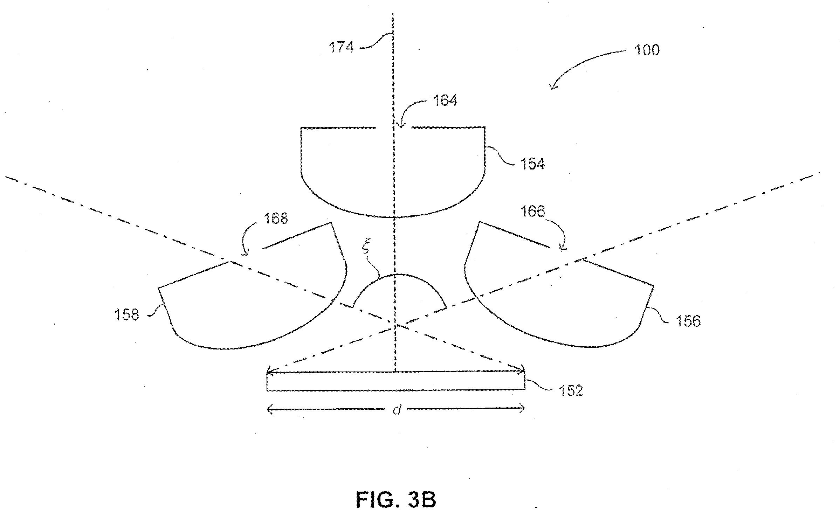

Reference is now made to Figures 3A and 3B, which are schematic illustrations of WFOV optical detector assembly, generally referenced 150, constructed and operative in accordance with another embodiment of the disclosed technique. Figure 3B is a side view of optical detector assembly 150. Optical detector assembly 150 includes an optical sensor 152 and optical receptors 154, 156, 158, 160 and 162. Optical receptors 154, 156, 158, 160 and 162 are spaced apart from each other. Each one of optical receptors 154, 156, 158, 160 and 162 includes an entrance pupil and a lens. Optical receptor 154 includes an entrance pupil 164, optical receptor 156 includes an entrance pupil 166, optical receptor 158 includes an entrance pupil 168, optical receptor 160 includes an entrance pupil 170 and optical receptor 162 includes an entrance pupil 172.

Optical receptors 154, 156, 158, 160 and 162 are optically coupled with optical sensor 152. The FOV $\xi$ (Figure 3B), of optical detector assembly 150 is increased relative to the FOV of a single optical receptor (e.g., optical receptor 106 in Figure 2B) without changing the size d of optical sensor 152 or the focal lengths of the lenses. As mentioned above optical receptors 154, 156, 158, 160 and 162 may be optical lenses. Alternatively, optical receptors 154, 156, 158, 160 and 162 may be replaced with pinholes. Optical detector 150 exhibits a directional response.

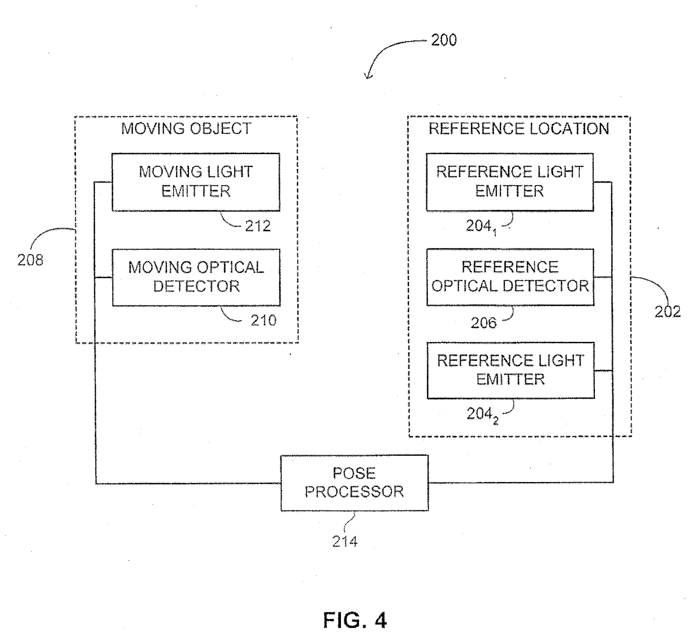

Reference is now made to Figure 4, which is a schematic illustration of an optical tracking system, generally referenced 200, for determining the pose (i.e., position and orientation) of a moving object 208 in a reference coordinate system in accordance with a further embodiment of the disclosed technique. System 200 includes a reference optical detector 206, reference light emitters $204_1$ and $204_2$, moving optical detector 210, a moving light emitter 212 and a pose processor 214. Either one of reference optical detector 206 or moving optical detector 210 may be a WFOV optical detector as described hereinabove in conjunction with Figures 2A and 2B or Figures 3A and 3B. Pose processor 214 is coupled with reference optical detector 206 and with moving optical detector 210. When reference light emitters $204_1$ and $204_2$ and moving light emitter 212 are light sources (e.g., LEDs), pose processor 214 is optionally coupled therewith. Reference optical detector 206 and reference light emitters $204_1$ and $204_2$ are situated at a known position 202 in a reference coordinate system (not shown). In general, reference optical detector 206 and moving optical detector 210 may be wired or wirelessly coupled with pose processor 214 and transmit information relating to the image or images acquired thereby over a wireless communication channel and employing a wireless communication protocol (e.g., Bluetooth or WiFi).

Moving optical detector 210 and moving light emitter 212 are attached to moving object 208. Moving light emitter 212 and reference light emitters $204_1$ and $204_2$ are, for example, Light Emitting Diodes (LEDs) emitting light at a desired spectral range (e.g., visible light, infrared). Each of reference optical detector 206 and moving optical detector 210 exhibit a directional response. Each of reference optical detector 206 and moving optical detector 210 include an optical sensor (not shown). The optical sensors are, for example, Charge Coupled Devices (CCDs), Complementary Metal Oxide Semiconductor (CMOS) sensor, a Position Sensitive Device (PSD) or a lateral photo-detector.

Reference light emitters $204_1$ and $204_2$ and moving light emitter 212 emit light either periodically (i.e., light pulses) or continuously. Reference optical detector 206 acquires an image or images of moving light emitter 212. Moving optical detector 210 acquires an image or images of reference light emitters $204_1$ and $204_2$. The term "acquires an image" refers herein to the exposure of the sensor in the detector to light and the accumulation of energy in the sensor pixels. Reference optical detector 206 and moving optical detector 210 provide information relating to the acquired image or images to pose processor 214.

According to one alternative, the information relating to the acquired image relates to a pre-processed image or a pre-processed portion of the image. In other words, reference optical detector 206 and moving optical detector 210 sample the energy values of at least a portion of the sensor pixels, and pre-process the sampled pixels (i.e., pre-process at least a portion of the image). This pre-processing includes, for example, filtering, segmenting (e.g., Binary Large Object - BLOB detection), scaling, rotating and the like. Alternatively or additionally, reference optical detector 206 and moving optical detector 210 provide information relating to objects in the image (i.e., as determined during segmentation). This information relates, for example, to the size, location, color, texture of the object and the like.

According to another alternative, the information relating to the acquired image refers to the sampled energy values of at least a portion of the senor pixels. In other words, reference optical detector 206 and moving optical detector 210 sample the energy values of at least a portion of the sensor pixels (i.e., at least a portion of the acquired image) and provide the sampled pixels to pose processor 214. Pose processor 214 pre-processes the sampled image (or the portion thereof).

When reference optical detector 206 and moving optical detector 210 acquire an image or images of light emitters 212, $204_1$ and $204_2$, the information relating to the acquired images include information relating to light emitters 212, $204_1$ and $204_2$. The information relating to light emitters 212, $204_1$ and $204_2$ is referred to herein as "representations" of the light emitters. These representations may be the sampled image or images or information relating to objects in the image associated with light emitters 212, $204_1$ and $204_2$. Pose processor 214 determines the pose of moving object 208 relative to the reference coordinate system according to the representations of light emitters 212, $204_1$ and $204_2$ provided thereto by reference optical detector 206 and moving optical detector 210.

In general to determine the position and orientation of moving object 208, pose processor 214 generates and solves at least six equations with six unknowns (e.g., three unknowns for position, the x, y and z coordinates and three unknowns for orientation, the azimuth elevation and roll angles). A representation of a light emitter is associated with two angles. For example, when the optical sensor is a CCD sensor, that CCD sensor is associated with a physical center. An imaginary line passing through this physical center, perpendicular to the sensor plane, defines the optical axis of the CCD sensor. Each pixel in CCD sensor is associated with a respective location on the CCD sensor, defined by a sensor 2D coordinates system in pixel units (e.g., a pixel located at coordinates [2;3] in the sensor 2D coordinates system is the pixel at the intersection of the second colon of pixels with the third row of pixels). Accordingly, each pixel is associated with a horizontal angle and a vertical angle from the optical axis of the sensor, related to the location of the pixel in the sensor 2D coordinate system. Consequently, each representation of a light emitter determined from an image acquired by the CCD sensor is also associated with a respective horizontal angle and a vertical angle from the center of the optical axis of the CCD sensor.

Thus, the representation of moving light emitter 212 determined from the image acquired by reference optical detector 206 is associated with two respective angles. Furthermore, each representation of each reference light emitter

$204_1$ and $204_2$ determined from the image acquired by moving optical detector 210 is also associated with two respective angles. Accordingly a total of six measurements of angles are acquired, along with the known spatial relationship (i.e., relative position) between reference light emitters $204_1$ and $204_2$ and optical detector 206, and the known spatial relationship between moving light emitter 212 and optical detector 210, define the above mentioned six equations with six unknowns. Pose processor, 214 solves these equations to determine the position and orientation of moving object 208 in reference coordinate system. When a single optical detector is employed, which acquires an image of at least three light emitters, the two angles associated with each representation along with the known spatial relationship between the light emitters define the above mentioned six equations with six unknowns.

It is noted that system 200 can determine the pose of moving object 208 as long as reference light emitters $204_1$ and $204_2$ are within the FOV *v* of moving optical detector 210 and as long as moving light emitter 212 is within the FOV of reference optical detector 206. It is further noted that when moving optical detector 210 is a WFOV optical detector, each optical receptor projects a respective representation of light emitters $204_1$ and $204_2$ on the optical sensor of moving optical detector 210.

Pose processor 214 associates the representations of light emitters $204_1$ and $204_2$ with the respective optical receptor, projecting these representations on the optical sensor. The first association of a representation and an optical receptor can be performed, for example, based on a low-accuracy tracking system such as an inertial tracker which provides a position and orientation. According to this example, the pose processor 214 determines a position and orientation of each possible association between a representation and an optical receptor and chooses the association in which the orientation is most similar to that determined by the low-accuracy tracker (e.g., an inertial tracker or a magnetic tracker). Alternatively, pose processor 214 associates the representations of light emitters $204_1$ and $204_2$ with the respective optical receptor, projecting these representations on the optical sensor, by determining a figure of merit for each representation. Pose processor 214 selects the association with the higher figure of merit. To that end, for each set of representations, processor 214 determines respective position and orientation of moving object 208. In the process of solving these equations (e.g., employing least squares), each solution, respective of each association between representations and a receptor, is associated with a respective residual error. Processor 214 selects the solution and thus the association with the smallest respective residual error (i.e., the figure of merit is the inverse, or an increasing function of the inverse of the residual error). In yet another alternative, when light emitters $204_1$ and $204_2$ are in the FOV of at least two of the optical receptors of moving optical detector 210, pose processor 214 associates the representations of light emitters $204_1$ and $204_2$ with the respective optical receptors according to the geometric configuration of the optical receptors. After associating each light emitter with a respective optical once, pose processor 214 tracks the representations of light emitters $204_1$ and $204_2$ on the optical sensor, and thus the association of the representations of light emitters with the respective optical receptor is maintained in the following cycles as further explained below.

When processing an image or images acquired by moving optical detector 210 and reference optical detector 206, it is not required to process the image in its entirety. Rather the pre-processing, performed either by pose processor 214 or by reference optical detector 206 and moving optical detector 210, is performed only over a predefined region of interest (ROI) within the image. The borders of the ROI are updated on a frame-by-frame basis. The ROI borders are determined according to the predicted locations of the representations of the light emitters in the acquired image, for example, based on the estimated relative orientation between the moving object and the reference location as determined by a low accuracy tracker. Alternatively, the ROI borders are determined according to the location of the representations of the light emitters in a previous image or images and an estimation of motion of moving optical detector 210. In general more than one ROI may be generated in order to track two or more groups emitters with minimal latency.

Although in Figure 4, the optical detector 210 is described as a moving optical detector and optical detector 206 as a reference optical detector, it is noted that, in general, these two detectors may exhibit relative motion there between (i.e., either one or both of optical detector 206 and optical detector 210 may move). The coordinate system in which the object is tracked may be that associated with the reference optical detector. Thus, an optical tracking system such as optical tracking system 200 may be employed for various tracking applications. For example, a tracking system similar to optical tracking system 200 may be employed in medical navigation applications, such as described below. It is further noted that any one of the light emitters described herein above and below may be a light source or a light reflector (e.g., ball reflector) which reflects light incident thereon (i.e., either ambient light, light from various light sources located at the vicinity of the light reflector or from a dedicated light source directing light toward the light reflector).

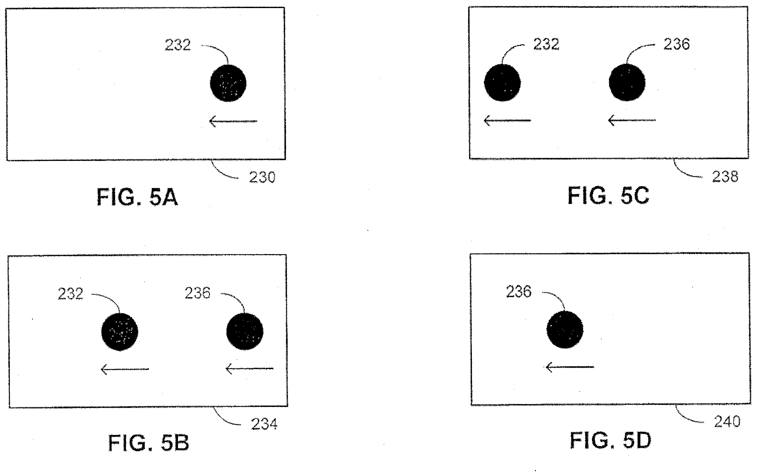

Reference is now made to Figures 5A, 5B, 5C and 5D which are schematic illustrations of images of a single light emitter acquired by a WFOV optical detector which includes only two adjacent optical receptors (not shown). In Figures 5A, 5B, 5C and 5D the WFOV optical detector moves from left to right relative to the light emitter. Consequently, emitter representation 232 and 236 of the light emitter (not shown), in images 230, 234, 238 and 240, move from right to left (i.e., relative to the image vertical axis), as designated by the arrow. In image 230 (Figure 5A), emitter representation 232 represents the light received from the light emitter and received by the first optical receptor. In images 234 and 238 (Figure 5B and 5C), emitter representations 232 and 236 represent the light received from the light emitter and received by both the optical receptors. In image 240 (Figure 5D), emitter representation 236 represents the light received from the light emitter and received by the second optical receptor. Thus, by tracking the representations of the light emitter, a pose processor (e.g., pose processor 214 in Figure 4) determines which optical receptor in the WFOV optical detector projects the light received from a light emitter. During initialization of the system or when the optical tracking system loses track of the moving object, the optical tracking system has no information relating to which one of the optical receptors projects light on the optical sensor. Therefore, the system determines the correct association using one of the above mentioned methods.

According to another embodiment of the disclosed technique, the spatial setup of the light emitters and the detectors enables the optical tracking system to determine the orientation of a moving object, in a reference coordinate system, without determining the position of the object. According to this spatial setup, a light emitter is placed at the entrance pupil of each optical receptor and emits light therefrom. Alternatively, a virtual representation of the light emitter can be created at the entrance pupil of the optical receptor (e.g., using beam splitters situated in front of the entrance pupil of the optical receptor). Consequently, the light emitter is perceived as emitting light from the entrance pupil of the optical receptor. In yet another alternative, two light emitters are placed such that the optical center of gravity thereof (e.g., the average position vector, in the reference coordinate system, of the two light emitters) is located at the entrance pupil of the optical receptor. Referring back to Figure 4, a virtual representation (not shown) of light emitter 212 is formed at the entrance pupils of the optical receptors of moving optical detector 210. Reference light emitters $204_1$ and $204_2$ are positioned such that the optical center of gravity thereof is located at the entrance pupil of the optical receptor of reference optical detector 206. Consequently orientation processor determines the orientation of moving object 208 without determining the position thereof.

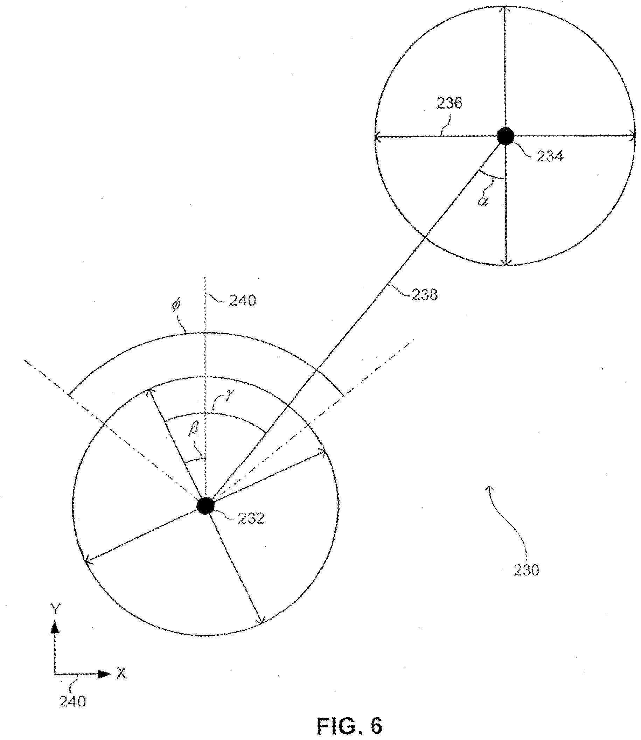

Reference is now made to Figure 6 which is an example for determining the horizontal orientation of a moving object without determining the position thereof in accordance with another embodiment of the disclosed technique and still referring back to Figure 4. It is noted that in exemplary Figure 6, the position of moving object 208 changes in the X, Y plane of two-dimensional (2D) coordinate system 240, and the orientation of moving object 208 may change only horizontally. It is further noted that the example brought herein is operative in either one of two cases. In the first case the light emitters emit light from the entrance pupil of the optical receptor of the optical detector. In the second case at least two light emitters are situated such the optical center of gravity thereof is located at the pupil of the optical detector. It is also noted that the roll angle is assumed to be zero.

Pose processor 214 determines the angle a, between the longitudinal axis 240 of reference coordinate system 236 and line 238 connecting entrance pupil 232 and entrance pupil 234 of moving optical detector 206 and reference optical detector 210 respectively. Pose processor 214 determines this angle $\alpha$ according to the location of a representation of moving light emitter 212 in an image acquired by reference optical detector 206. For example, when the optical sensor of reference optical detector 206 is a CCD sensor, each pixel in the CCD is associated with an angular step. Thus, angle $\alpha$ is that angular step multiplied by the number of horizontal pixels counted from the optical center of the CCD. It is noted that moving light emitter 212 emits light from the entrance pupil of the optical receptor of moving optical detector 210 (e.g., via a beam splitter).

Pose processor 214 determines the angle y, between the optical axis of the moving optical detector 210 and Line 238 connecting entrance pupil 232 and entrance pupil 234. Pose processor 214 determines the angle $\gamma$ according to the location of the representations of reference light emitters $204_1$ and $204_2$ on an image acquired by moving optical detector 210. The optical center of gravity of reference light emitters $204_1$ and $204_2$ is situated at the entrance pupil of the optical receptor of reference optical detector 206.

Pose processor 214 determines the horizontal orientation of moving object 208 by determine the angle between optical axis of the moving optical detector 210 and longitudinal axis 240, designated by the angle $\beta$. Orientation processor determines the angle $\beta$ according to:

$$\beta = \gamma - \alpha \qquad\qquad (1)$$

Thus, according to the example brought hereinabove, orientation processor 214 determines the horizontal orientation angle of moving object 208 without determining the position thereof.

As mentioned above, the exemplary method described in conjunction with Figure 6 is operative when the light emitters emit light from the entrance pupil of the optical receptor and the roll angle is zero. The method may also be operative when the roll angle is substantially small, resulting in an approximation of the azimuth and elevation angles. Alternatively, the method described in conjunction with Figure 6 is operative in situations wherein the roll angle is known. For example, the two light emitters are situated such that the optical center of gravity thereof is located at that entrance pupil (i.e., the roll angle is known according to the representations of the two light emitters on the opposite optical sensor). In yet another example, the roll angle is known from gravitational tilt sensors. For the exemplary method of Figure 6 to be operative with the WFOV optical detector described in conjunction with Figures 2A, 2B, 3A and 3B, a light emitter is associated with a respective one entrance pupil described therein, and emits light therefrom. Alternatively, at least a pair of light emitters is associated with a respective one entrance pupil and the optical center of gravity thereof is located at that respective entrance pupil. Furthermore, when light is determined as entering through an entrance pupil or pupils, associated with the light emitter or light emitters, the optical tracking system relates to the light emitted by this light emitter or these light emitters (e.g., by selecting the representation of the light emitter or emitters on the opposite optical detector or by enabling these light emitters).

The method described in conjunction with Figure 6 may be applied when moving object 208 moves in three-dimensions (3D). Accordingly, the orientation of moving object 208 may change in the horizontal, vertical and roll directions. Equation (1) may be applied in both the horizontal and vertical cases. The results of equation (1) are a horizontal orientation angle and a vertical orientation angle. The azimuth and elevation are approximated according to the horizontal orientation, vertical orientation and roll angles. The roll angle may be determined, for example, as mentioned above, according to the representations of the two light emitters on the opposite optical sensor.

According to a further embodiment of the disclosed technique, a reflective surface replaces the reference detector. Thus, the optical tracking system determines the orientation of a moving object, in a reference coordinate system, without determining the position of the moving object. According to this configuration, the optical tracking system includes a light emitter attached to the moving object and a reflective surface situated at a known position in the reference coordinate system. A reflection of the moving light emitter is formed on the fixed reflective surface. When the roll angle is substantially small, the reflection of the moving light emitter is affected only by the change in the azimuth and the elevation angles of the moving object (i.e., yaw and pitch), and not by the translation of the moving object (i.e., there is no parallax). Consequently, the optical tracking system determines the two angle orientation of the moving object according to an image of the reflection of the moving light emitter, acquired by moving light detector. For determining the roll angle (i.e., when accurate values of the azimuth and elevation angles are required), the reflective surface may include additional emitters at the vicinity thereof.

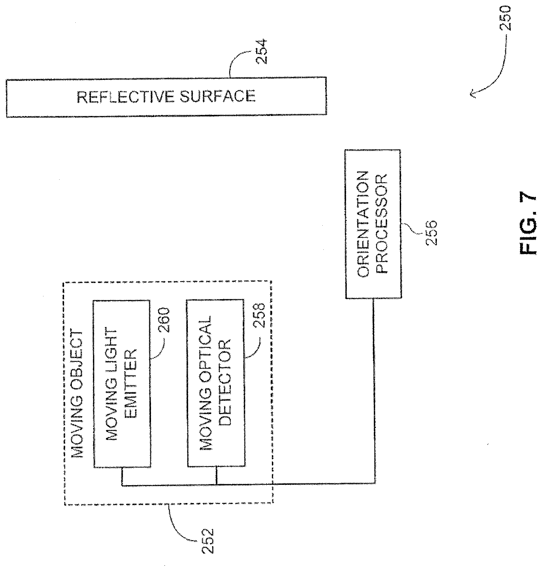

Reference is now made to Figure 7, which is a schematic illustration of an optical tracking system, generally reference 250, constructed and operative in accordance with a further embodiment of the disclosed technique. System 250 includes a moving object 252, a reflective surface 254 and an orientation processor 256. Moving object 252 includes a moving optical detector 258 and light emitter 260. Moving optical detector 258 may be a WFOV optical detector as described hereinabove in conjunction with Figures 2A and 2B or Figures 3A and 3B. Moving optical detector 258 and light emitter 260 are all coupled with orientation processor 256. Light emitter 260 emits light toward reflective surface 254. Reflective surface 254 reflects the light back toward moving WFOV optical detector 258. Reflective surface 254 is, for example, a flat mirror. Reflective surface 254 may further be any surface reflecting the light emitted by Light emitter 260 such as a computer screen, a television screen, a vehicle or aircraft windshield and the like. Reflective surface 254 may be a wavelength selective reflective surface (i.e., reflective surface 254 reflects radiation within a range of wavelengths only). Moving optical detector 258 acquires an image of the reflection of moving light emitter 260. Orientation processor 256 determines the orientation of moving object 252 according to the acquired image of the reflection of light emitter 260. Orientation processor 256 determines the azimuth and elevation angles of the moving object according to the (x, y) location of the light emitter in the image (i.e., when the roll angle is substantially small). However, system 250 described hereinabove in conjunction with Figure 7, determines the azimuth and elevation angles only. When system 250 is required to determine the roll angle as well, two additional light emitters are fixed, for example, at either side of reflective surface 254. System 250 determines the roll angle according to the position of the two light emitters in the image. Alternatively, a single light emitter of a shape exhibiting rotational asymmetry around an axis normal to the object plane (i.e., where the light emitter is located), within a desired range of roll angles (e.g., an ellipse, an isosceles triangle) is fixed at the vicinity of the reflective surface.

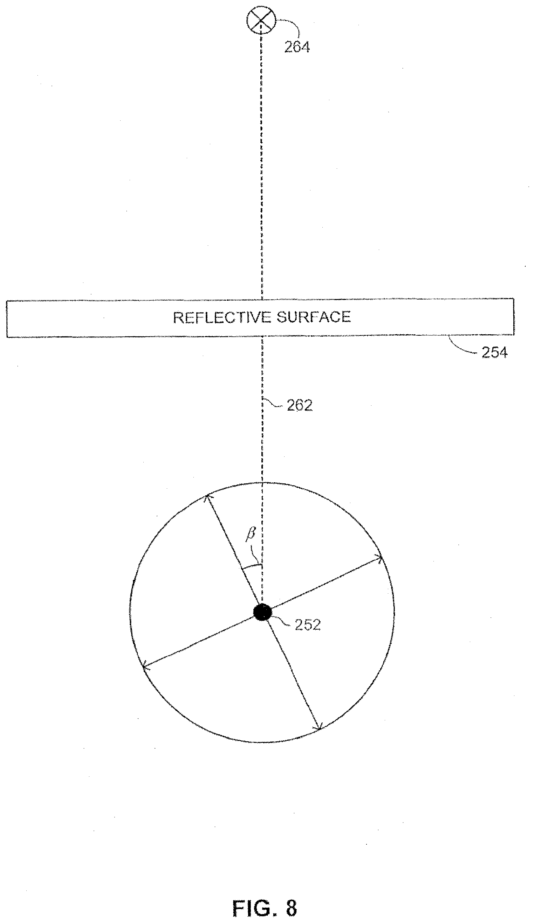

Reference is now made to Figure 8, which is a schematic illustration of a two-dimensional example for determining the orientation of a moving object without determining the position thereof in accordance with another embodiment of the disclosed technique and referring back to Figure 6. Orientation processor 256 determines the orientation of moving object 252, designated by the angle $\beta$, by determining in which angular section of the observed scene mirror image 264 of moving light emitter 260 is situated (i.e., by tracking light incident on the sensor of moving optical detector 258). Orientation processor 256 determines the angle $\beta$ further, according to the location of the projection of mirror image 264 of moving light emitter 260 on moving optical detector 258. As in the example brought hereinabove, when the optical sensor of moving optical detector 258 is a CCD sensor, each pixel in the CCD is associated with an angular step. Thus angle $\beta$ is that angular step multiplied by the number of pixels counted from the optical center of the CCD sensor. As mentioned above, the angle $\beta$ is determined when the roll angle is substantially small.

As mentioned above, a WFOV optical tracking system according to the disclosed technique may be employed in various medical navigation applications and scenarios. For example, a WFOV optical tracking system according to the disclosed technique may be employed for tracking the position and orientation of a medical tool and presenting a real-time representation of the medical tool on an image of a patient. The medical tool may be a hand held tool or a movable tool such as a needle, a real-time imager (e.g., a real-time ultrasound imager, a real time X-ray imager located on a C-arm), a stylus, a surgical knife, a catheter and the like. In general, the medical WFOV optical tracking system according to the disclosed technique, determines the position and orientation of at least one target object in a reference coordinate system. Such a system generally includes at least three light emitters and at least one optical detector which can be a WFOV optical detector. The WFOV optical detector is similar to as described above in conjunction with Figures 2A, 2B, 3A and 3B. The reference location is associated with a reference coordinate system. Each optical detector and each light emitter is adapted to be attached to a respective one of at least one target object and a reference location. Each optical detector acquires an image of the light emitter or light emitters within the field of view thereof. Furthermore, each of the light emitters is within the field of view of at least one of the optical detector or detectors. A processor determines the position and orientation of the target object in the reference coordinate system, according to the representations of the light emitters (i.e., as determined either by the optical detector which acquired the image or by the processor both as explained above in conjunction with Figure 4) similar to as described above in conjunction with Figure 4.

The above mentioned medical WFOV optical tracking system may exhibit various configurations. According to one exemplary configuration, the medical WFOV optical tracking system includes a WFOV optical detector and at least three light emitters. The WFOV optical detector is attached to a target object and the three light emitters are attached to the reference location or vice versa. According to another exemplary configuration, the medical WFOV optical tracking system includes a WFOV optical detector, another optical detector and at least three light emitters. The WFOV optical detector is attached to the target object and the other optical detector is attached to the reference location (or vice versa). One light emitter is attached to target object and two light emitters are attached to the reference location (or vice versa).

In general there can be more than one target object. Each pair consisting of the reference location and a target object is associated with at least three light emitters, each attached to either the target object or the reference location employed for determining the position and orientation of the target object. The above mentioned target object or objects and the reference location are respective elements in a tuple including at least two elements from a group consisting of a display (e.g., Head Mounted Display - HMD) further explained below, a patient body location, a medical tool, a physician body location, and a fixed position. Following are various examples for such a tuple of elements including at least two elements: {display, medical tool}, {fixed position, medical tool}, {display, medical tool, medical tool}, {display, medical tool, fixed position}, {display, patient body location, medical tool}, {fixed position, medical tool, medical tool}, {fixed position, patient body location, medical tool}, {patient body location, medical tool, physician body location}, {fixed position, patient body location, medical tool, physician body location}, {display, fixed position, patient body location, medical tool}, {display, patient body location, medical tool, medical tool}, {tool, tool}, and {patient body location, patient body location}. The tuple {tool, tool} refers to two different medical tools (i.e., the target object or objects and the reference location are all medical tools). The tuple {body part of patient, body part of patient} refers to two different patient body locations (e.g., thigh and leg). Each optical detector and each light emitter is attached to a respective one of the elements. Furthermore, each element may be designated as the reference location according to particular needs and the remaining elements are designated as target objects. The above mentioned fixed position may be for example, the wall or the ceiling of an operating room. Alternatively, the fixed position may be a mechanical support (e.g., a tripod, a mechanical arm), which is stationary during part of the medical procedure and may be moved between parts of the procedure. Furthermore, each of the above elements may be associated with a respective coordinate system. These respective coordinate systems may be registered with each other and with the reference coordinate system.

As mentioned above, medical optical tracking system according to the disclosed technique may include a display. The display displays a model representing information relating to the patient. The display may also display real-time image of the patient, such as real-time ultrasound images, real-time X-ray images (e.g., acquired with X-ray imager located on a C-Arm), laparoscopy images and real-time MRI images. The display may further display representations relating to medical tools as well as navigational information (e.g., marks representing target locations or the trajectory of medical tool). The display may be, for example, a 2D or 3D screen display (e.g., LED display, LCD display, plasma display or Cathode Ray Tube - CRT display), a hand-held display (e.g., a tablet computer), an image overlay device, or a Head Mounted Display (HMD) as further explained below. The model is, for example, a two dimensional (2D) or a three dimensional (3D) image of the patient, for example, 2D X-Ray image, a pre-acquired CT model or MRI model. Alternatively, the model may be a symbolic model or a virtual model representing the body of the patient or various regions thereof (e.g., the heart, the brain or the uterus, tumor within the body, the circulatory system or parts thereof). The model and the navigational information is associated with the reference coordinate system. When the model is an image, the coordinate system associated with the image is registered with the reference coordinate system as further explained below.

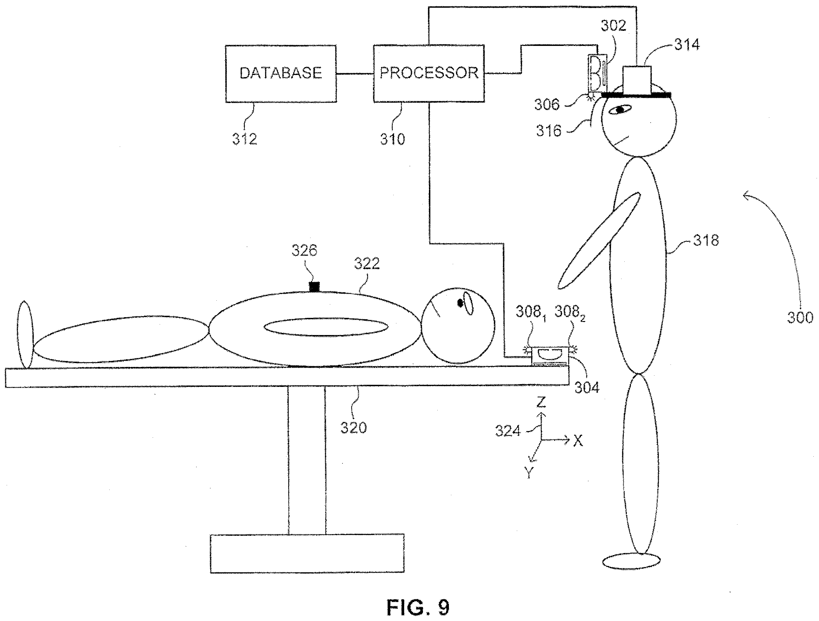

The description herein below, in conjunction with Figures 9, 10, 11, 12, 13, 14, 15 and 16 present various examples of several of the above described medical WFOV optical tracking systems. According to a first example, the medical WFOV optical tracking system according to the disclosed technique may be employed for presenting a model of a patient to a physician at an orientation and scale corresponding to the point of view of the physician. Reference is now made to Figure 9, which is a schematic illustration of an exemplary medical WFOV tracking system, generally reference 300, in accordance with a further embodiment of the disclosed technique. System 300 includes a WFOV optical detector 302, another optical detector 304, at least one light emitter 306, at least two other light emitters $308_1$ and $308_2$, a processor 310, a database 312 and a display such as Head Mounted Display (HMD) 314. HMD 314 includes a visor 316. Alternatively HMD 314 may be in the form of a near-to-eye display (i.e., displays located close to the eye such as Google glass and the like).

Processor 310 is coupled with database 312, WFOV optical detector 302, HMD 314, optical detector 304. When light emitter 306, light emitters $308_1$ and $308_2$ are light sources such as LEDs, processor 310 is optionally coupled with light emitter 306 and with light emitters $308_1$ and $308_2$. Light emitter 306 is attached to WFOV optical detector 308 and both are attached to HMD 314. HMD 314 along with WFOV optical detector 308 and light emitter 306 are donned by a physician 318. Alternatively, light emitter 306 may be directly attached to HMD 314. Optical detector 304 is firmly attached to a treatment bed 320 on which a patient 322 lies. System 300 is associated with a reference coordinate system 324 which, in this case, is also the coordinate system associated with optical detector 304. However, reference coordinate system 324 may alternatively be the reference coordinate system of, for example, HMD 314.

Database 312 stores a model of the body of patient 322 or parts thereof (e.g., the head, the torso). This model may be 3D model such as a symbolic or a virtual model. Alternatively, the model is a 3D model such as a Computer Tomography (CT) model or a Magnetic Resonance Imaging (MRI) model. The model is associated with reference coordinate system 324. When the model is an image, the coordinate system associate with the image is registered with reference coordinate system 324 (i.e., each position in the coordinate system associate with the image has a corresponding position in reference coordinate system 324). This registration is achieved, for example, by employing image processing techniques to determine the position of a fiducials, such as fiducial 326, which is also visible in the image, in the coordinate system associate with the 3D image. Then, the location of fiducial 326 is determined in reference coordinate system 324, for example by employing a stylus fitted with a WFOV optical detector as further explained below. Thus, a correspondence between the location of the fiducial in coordinate system 324 and in the coordinate system associated with the image. To register, for example, two 3D coordinate systems, the position and orientation of at least three common distinct features should be determined in both coordinate systems. The distinct features may also be distinct features on the body of patient 322, such as the nose bridge, the medial cantus or the tragus. Processor 310 then determines the transformation between the two coordinate systems.

One exemplary use of system 300 may be for marking incision marks on a patient employing a 3D image of the patient. To that end, patient 322 lies on treatment bed 320. WFOV optical detector 302 and optical detector 304 acquire an image or images of the light emitters within the FOV thereof. In Figure 9, WFOV optical detector 302 acquires an image or images of light emitters $308_1$ and $308_2$ and optical detector 304 acquires an image of light emitter 306. Processor 310 determines the position and orientation of WFOV optical detector 302 relative to optical detector 304, and consequently in reference coordinate system 324, according to the representation of light emitter $308_1$ and $308_2$ and light emitter 306 (i.e., as determined from the acquired images either by WFOV optical detector 302 and optical detector 304 respectively or by processor 310). Thus processor 310 determines the position and orientation of the head of physician 318 in reference coordinate system 324. Since the 3D image is registered with reference coordinate 324, processor 310 renders the 3D image such that HMD 314 displays the 3D image on visor 316 at the scale and orientation corresponding to the determined position and orientation (i.e., the point of view) of physician 318. Physician 318 can view internal parts of patient 322 and, for example, mark incision marks on patient 322 which are suitable for the required procedure. It is noted that, when HMD 314 displays an image on visor 316, processor 310 may further have to adjust the orientation of the image to account for the angle between the optical center of the visor and optical detector 316 attached to HMD 314. Since WFOV optical detector 302 is attached to HMD 314, this angle is fixed and needs to be determined only once.

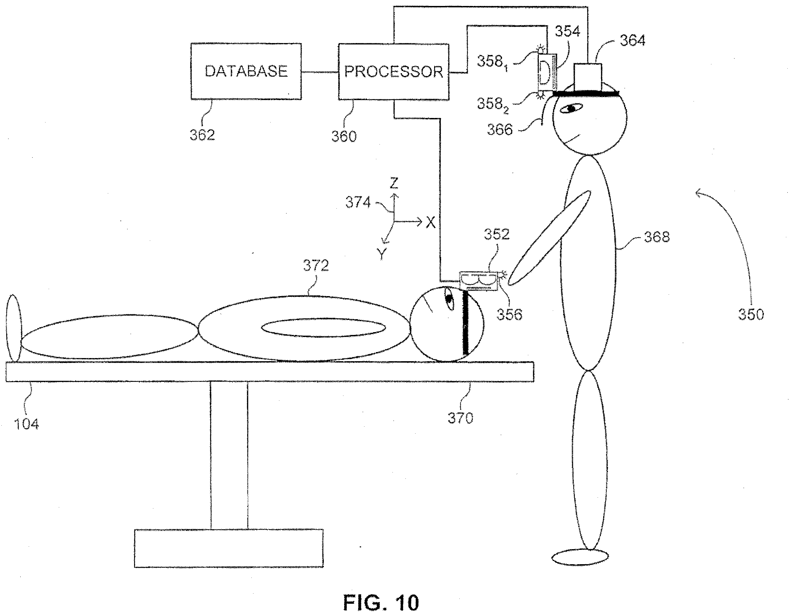

In the above description of system 300, the patient must remain stationary for the superposition of the model on the patient to be correctly maintained. However, according to another example, a detector is placed on the patient and the relative position and orientation of between the two detectors (i.e., and thus between the head of the physician and the patient) is determined. Furthermore, similar to as described above in conjunction with Figure 9, the model is associated with the reference coordinate system. When the model is a 3D image, the coordinate system associated with the 3D image is registered with the coordinate system associated with the detector placed on the patient. Thus, the patient may move and the 3D image shall move therewith, thereby maintaining the correct perspective of the image for the physician. Reference is now made to Figure 10, which is a schematic illustration of an exemplary medical WFOV tracking system, generally reference 350, in accordance with another embodiment of the disclosed technique. System 350 includes a WFOV optical detector 352, another optical detector 354, at least one light emitter 356, at least two other light emitters $358_1$ and $358_2$, a processor 360, a database 362 and a display such as HMD 364. HMD 364 includes a visor 366. HMD 364 may also be in the form of goggles.

Processor 360 is coupled with database 362, WFOV optical detector 352, HMD 364 and with optical detector 354. When light emitter 356, light emitters $358_1$ and $358_2$ are LEDs processor 360 is optionally coupled with light emitter 356 and with light emitters $358_1$ and $358_2$. Light emitter 356 is attached to WFOV optical detector 352 and both are attached to a body location of patient 372 (i.e., either directly or attached to a fixture which is attached to a body location of patient 372, such as the head, a limb or the jaw). HMD 364 along with optical detector 354 and light emitters light emitter $358_1$ and $358_2$ are attached to the head of a physician 368. Alternatively, light emitters $358_1$ and $358_2$ may be directly attached to HMD 364. System 350 is associated with a reference coordinate system 374 which, in this case, is also the coordinate system associated with WFOV optical detector 352.

Similar to database 312 (Figure 9), Database 362 stores a model patient 372 or of a part thereof. When the model is an image (i.e., a 2D image or a 3D image), the coordinate system associate with the image is registered with reference coordinate system 374. Similar to system 300 (Figure 9), system 350 may be employed, for example, for marking incision marks on a patient employing a 3D image of the patient. To that end, patient 372 lies on treatment bed 370. WFOV optical detector 352 and optical detector 354 acquire an image or images of the light emitters within the FOV thereof.

In Figure 10, WFOV optical detector 352 acquires an image or images of light emitters $358_1$ and $358_2$ and optical detector acquire 354 acquire an image of light emitter 356. Processor 360 determines the relative position and orientation of WFOV optical detector 352 relative to optical detector 354, and consequently in reference coordinate system 374 (i.e., the relative position and orientation between optical detector 354 and WFOV optical detector 352 and thus between patient 372 and the head of physician 368) according to the representation of light emitter 356 and the representations of light emitters $358_1$ and $358_2$ (i.e., as determined from the acquired images either by optical detector 354 and optical detector 352 respectively or by processor 360). Similar to as described above in conjunction with Figure 9, since the 3D image is registered with reference coordinate 374, processor 360 renders the 3D image such that HMD 364 displays the 3D image on visor 366 at the scale and orientation corresponding to the determined relative position and orientation between WFOV optical detector 352 (i.e., the patient) and optical detector 354 (i.e., the physician). Thus, in the event that either patient 372 and physician 368 move one with respect to the other, the perspective of 3D image presented to physician 368 shall be adjusted accordingly and shall appear superimposed on the body of patient 372. Physician 368 can view internal parts of patient 372 and mark incision marks on patient 372 which are suitable for the required procedure. Similar to as mentioned with regards to system 300 (Figure 9), when HMD 364 displays an image on visor 366, processor 310 may further have to adjust the orientation of the image to account for the angle between the optical center of the visor and optical detector 354 attached to HMD 364. Since optical detector 354 is attached to HMD 364, this angle is fixed and needs to be determined only once. One particular advantage of the medical WFOV optical tracking system described in conjunction with Figure 10 is the freedom of motion provided for physician 368 to move around patient 372 without losing track.

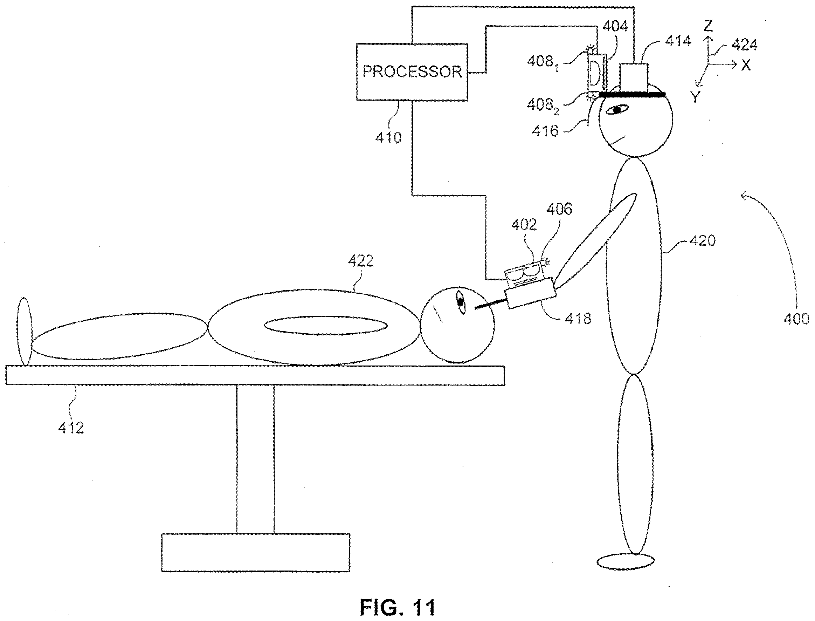

The medical WFOV optical tracking system according a further embodiment of the disclosed technique may be employed for tracking the position and orientation of a medical tool and for guiding a medical tool during a medical procedure. Reference is now made to Figure 11, which is a schematic illustration of an exemplary medical WFOV tracking system, generally reference 400, in accordance with a further embodiment of the disclosed technique. System 400 is used for tracking a medical tool in a reference coordinate system. System 400 includes a WFOV optical detector 402, another optical detector 404, at least one light emitter 406, at least two other light emitters $408_1$ and $408_2$, a processor 410 and a display such as HMD 414. HMD 414 includes a visor 416. HMD 414 may also be in the form of near-eye-display.

Processor 410 is coupled wit detector 402, HMD 414, optical detector 404. When light emitter 406 and light emitters $408_1$ and $408_2$ are LEDs, processor 310 is optionally coupled with light emitter 406 and light emitters $408_1$ and $408_2$. Light emitter 406 is attached to WFOV optical detector 402 and both are attached to medical tool 418. Light emitter $408_1$ and $408_2$ are attached to optical detector 404 and both are attached to HMD 414. HMD 414 along with optical detector 404 and light emitters $408_1$ and $408_2$ are attached to the head of a physician 420. Alternatively, light emitters $408_1$ and $408_2$ may be directly attached to HMD 414. System 400 is associated with a reference coordinate system 424 which, in this case, is also the coordinate system associated with optical detector 404. Medical tool 418 is, for example, an ultrasound imager, a medical knife, a catheter, a laparoscope, an endoscope or a medical stylus used by a physician 420 during a procedure conducted on a patient 422. Medical tool 418 is tracked in a reference coordinate system 424 associated with system 400. In Figure 10 reference coordinate system 424 is also the coordinate system associated with detector 404.

System 400 may be employed for tracking medical tool 418 even when part thereof may be concealed. Furthermore, System 400 may also be employed for presenting data acquired by medical tool 418 at the location from which that data was acquired. For example, when medical tool 418 is a medical needle (e.g., a biopsy needle) inserted into patient 422 lying on treatment bed 412, portions of medical tool 418 may be obscured to physician 400.

To that end WFOV optical detector 402 and optical detector 404 acquire an image or images of the light emitters within the FOV thereof. In Figure 11, WFOV optical detector 402 acquires an image or images of light emitters $408_1$ and $408_2$ and optical detector acquire 404 acquire an image of light emitter 406. Processor 410 determines the relative position and orientation between WFOV optical detector 352 (i.e., the tool) and optical detector 354 (i.e., the physician) according to representations of light emitters $408_1$ and $408_2$ and the representation of light emitter 406 (i.e., as determined from the acquired images either by WFOV optical detector 402 and optical detector 404 respectively or by processor 510). Furthermore, processor 410 constructs a visual representation (not shown) of the medical tool 418 or at least of the obscured portion of thereof, for example according to known dimensions of medical tool 418 and the known spatial relationship between WFOV optical detector 402 and medical tool 418. The spatial relationship between WFOV optical detector 402 and medical tool 418 is determined, for example, during the manufacturing process thereof. Medical tool 418, along with the visual representation thereof, is presented by HMD 414 on visor 416 to physician 420. Accordingly, physician 420 can view the location of the obscured portion of medical tool 418 within the body of patient 422. When medical tool 418 is an ultrasound imager, the image produced by the ultrasound imager may be displayed to physician 420 superimposed on the location in the body of patient 422 at which the image was acquired. Similar to as mentioned with regards to system 300 (Figure 9), when HMD 414 displays an image on visor 416, processor 410 may further have to adjust the orientation of the image to account for the angle between the optical center of the visor and optical detector 404 attached to HMD 414. Since optical detector 404 is attached to HMD 414, this angle is fixed and needs to be determined only once. One particular advantage of the medical WFOV optical tracking system described in conjunction with Figure 11 is the freedom of motion provided for physician 368 to move tool 418 to various positions and orientations without losing track.

According to another example, a medical WFOV optical tracking system may be employed for tracking the position and orientation of a medical tool in cases where the tool is obscured even when the patient moves. Additionally or alternatively, the medical WFOV optical tracking system according to the disclosed technique may further be employed for tracking the position and orientation of a medical tool, superimposed on a model (i.e., a symbolic or virtual model, or a 2D or a 3D image) of the patient. Thus, the physician may view and guide the medical tool toward a target location within the patient.

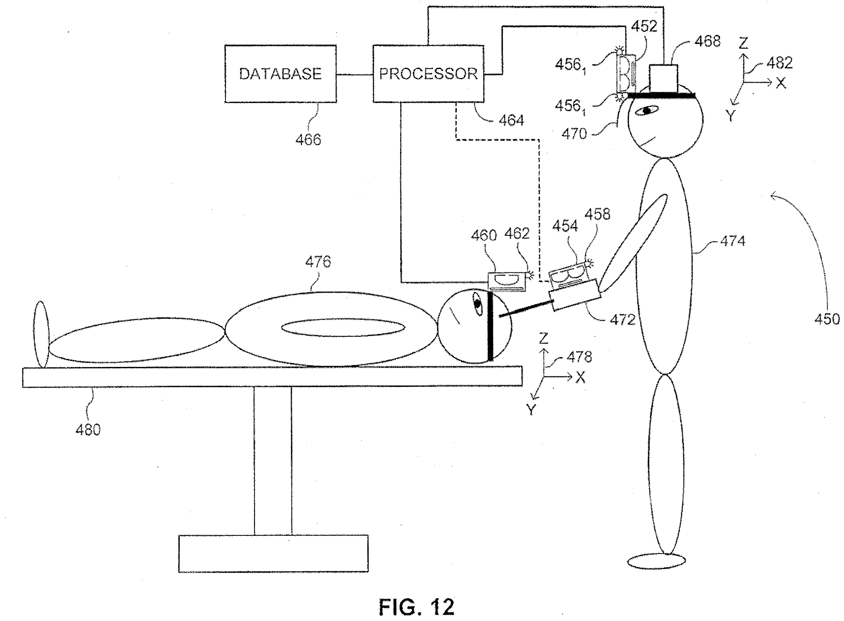

Reference is now made to Figure 12, which is a schematic illustration of an exemplary medical WFOV tracking system, generally reference 450, in accordance with another embodiment of the disclosed technique. System 450 is used for tracking a medical tool in a reference coordinate system, superimposed on a model of a patient. System 450 includes a first WFOV optical detector 452, a second WFOV optical detector 454, another optical detector 460, two light emitters $456_1$ and $456_2$, first light emitter 458 and second light emitter 462. System 450 further includes a processor 464, a database 466 and a display such as HMD 468. HMD 468 includes a visor 470. HMD 468 may also be in the form of near-eye-display.

Processor 464 is coupled with database 466, first WFOV optical detector 452, HMD 414, optical detector 460 and. Processor 464 is further wirelessly coupled with second WFOV optical detector 454 as indicated by the dashed line in Figure 12. When light emitters $456_1$ and $456_2$ and light emitter 462 are LEDs, processor 464 is optionally coupled therewith. When light emitter 458 is a LED, processor 464 is also optionally wirelessly coupled therewith. HMD 468 along with first WFOV optical detector 452 and light emitter 456 is donned by a physician 474. Light emitter 458 is attached to second WFOV optical detector 454 and both are attached to medical tool 472. Light emitter 460 is attached to optical detector 460 and all are attached to the head of patient 476 (i.e., the patient body location is the head) lying on treatment bed 480. System 400 is associated with a reference coordinate system 478 which, in this case, is also the coordinate system associated with optical detector 460. Furthermore, HMD 468 is associated with a respective coordinate system 482. Similar to medical tool 418 (Figure 11), medical tool 472 is, for example, an ultrasound imager, a medical knife, a catheter, a laparoscope or a medical stylus used by a physician 474 during a procedure conducted on a patient 476.

System 450 may be employed for tracking medical tool 472 by superimposing a representation of medical tool 472 on a model (i.e., a symbolic or virtual model or a 3D image) of patient 476. Furthermore, similar to system 400 described above in conjunction with Figure 11, system 450 may also be employed for presenting data acquired by medical tool 472 at the location from which that data was acquired. To that end, the coordinate system of the model is associated with reference coordinate system 478. When the model is an image of the patient, the coordinate system of the image is registered with reference coordinate system 478 (e.g., as described above in conjunction with Figure 9).

First WFOV optical detector 452, second WFOV optical detector 454 and optical detector 456 all acquire an image or images of the light emitters within the FOV thereof. In Figure 12, first WFOV optical detector 452 acquires an image or images of first light emitters 458 and of second light emitter 462. Second WFOV optical detector 454 and optical detector 460 both acquire an image or images of light emitters $456_1$ and $456_2$. Furthermore, second WFOV optical detector 454 may also acquire an image of light emitters 462 and optical detector 460 may also acquire an image of light emitter 458.

Processor 464 synchronizes the operation of first WFOV optical detector 452, of second WFOV optical detector 454, of optical detector 460, of light emitters $456_1$ and $456_2$, of light emitter 458 and of light emitter 462 such that the time period when first WFOV optical detector 452 acquires an image of light emitter 458 and second WFOV optical detector 454 acquires an image of light emitters $456_1$ and $456_2$, does not overlap with the time period when first WFOV optical detector 452 acquires an image of light emitter 462 and optical detector 460 acquires an image of light emitters $456_1$ and $456_2$. In other words, the operation of the pair of detectors including first WFOV optical detector 452 and second WFOV optical detector 454 and of light emitters $456_1$, $456_2$ and 458 is mutually exclusive in time with respect to the operation of the pair of detectors including first WFOV optical detector 452 and optical detector 460 and of light emitters $456_1$, $456_2$ and 460.

Processor 464 determines the position and orientation of first WFOV optical detector 452 in reference coordinate system 478 and consequently of HMD 468 relative to the head of patient 476, according to the representation of light emitter 462 and the representations of light emitters $456_1$ and $456_2$ (i.e., as determined either by first WFOV optical detector 452 and optical detector 460 respectively or by processor 464).. Also, processor 464 determines the position and orientation of optical detector 454 in coordinate system 482 respective of HMD 468 and consequently the position and orientation of medical tool 472 relative to HMD 468, according to the representation of light emitter 458 and the representations of light emitters $456_1$ and $456_2$ (i.e., as determined by either first WFOV optical detector 452 and second optical detector 454 respectively or by processor 464). Processor 464 may further synchronize second WFOV optical detector 454 and optical detector 460 with light emitters 458 and 462, and employ the representations of light emitter 462 acquired by second WFOV optical detector 454 and of light emitter 458 acquired by optical detector 460 to verify the correctness of the determined position and orientation of second WFOV optical detector 454 relative to optical detector 460.

According to the determined relative positions and orientations between medical tool 472, HMD 468 and patient 476, processor 464 may render the model of patient 476 in the correct perspective and provide the rendered model to HMD 468 and optionally, superimpose a representation of medical tool 472, at the corresponding position and orientation relative to the HMD 468. Furthermore, processor 464 may superimpose navigational information on the model. This navigational information is, for example, a mark representing a target location, a line representing the trajectory (including projected trajectory) of medical tool 472, marks representing the fiducial markers used for the registration. Processor provides HMD 468 with the rendered 3D image superimposed with a representation of medical tool 472 and with the navigational information. HMD 468 displays this rendered and superimposed image to physician 474 on visor 470. Additionally, since processor 464 determines the relative position and orientations between first WFOV optical detector 452, second WFOV optical detector 454 and optical detector 460, even when patient 476 moves, the model, the representation of medical tool 472 and the navigation information shall be adjusted to the new point of view of physician 474. HMD 468 may display only one of the model of a patient, representation of medical tool 472 and navigational information or any pair thereof.

Similar to as mentioned with regards to system 300 (Figure 9), when HMD 468 displays an image on visor 470, processor 464 may further have to adjust the orientation of the presented image to account for the angle between the optical center of the visor and fist WFOV optical detector 452 attached to HMD 468. Since first WFOV optical detector 452 is attached to HMD 468, this displacement is fixed and needs to be determined only once. It is also noted that system 450 may be modified to include two or more WFOV optical detectors attached to any of HMD 468 or medical tool 472, thus further increasing the FOV of tracking system 450. Similarly, system 450 may be modified to include two or more optical detectors attached to the patient. One particular advantage of the medical WFOV optical tracking system described in conjunction with Figure 12 is the freedom of motion provided for physician 474 to move tool 472 to various positions and orientations and for physician 474 to move around patient 476 without losing track.

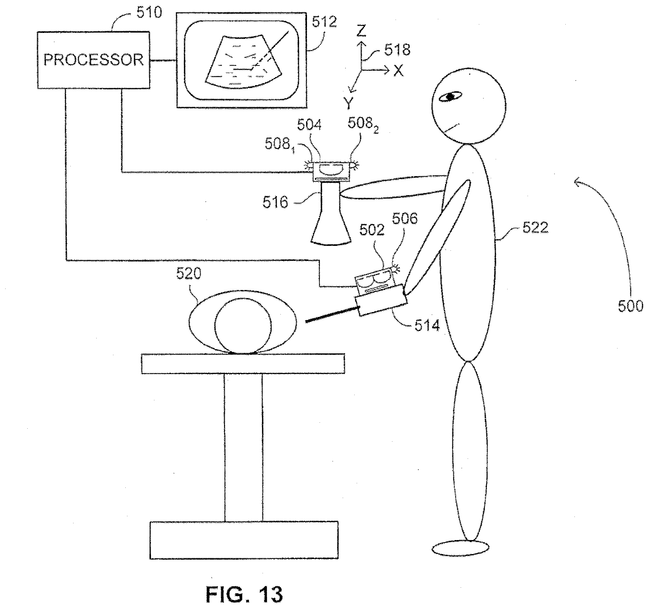

Reference is now made to Figure 13, which is a schematic illustration of an exemplary medical WFOV tracking system, generally reference 500, in accordance with a further embodiment of the disclosed technique. System 500 is employed for tracking one medical tool with respect to another medical tool. System 500 includes a WFOV optical detector 502, another optical detector 504, at least one first light emitter 506, and at least two other light emitters $508_1$ and $508_2$. System 450 further includes a processor 510 and a display 512.

Processor 510 is coupled with WFOV optical detector 502, with second optical detector 504. When first light emitter 506 and two other light emitters $508_1$ and $508_2$ are LEDs, processor 510 is optionally coupled therewith. Light emitter 506 is attached to WFOV optical detector 502 and both are attached to a first medical tool 512 which, in Figure 13 is exemplified as a needle (e.g., a biopsy needle or an amniocentesis needle). Light emitters $508_1$ and $508_2$ are attached of second optical detector 504 and all are attached to a second medical tool 514. Second medical tool 514 may be, for example, any real-time imaging device which, in Figure 13 is exemplified as an ultrasound imager. However, second medical tool 514 may alternatively be a laparoscopy camera, an endoscope, an X-ray imager located on a C-arm or a real-time MRI imager. System 500 is associated with a reference coordinate system 518 which, in this case, is also the coordinate system associated with optical detector 504. With regards to the above examples of real-time imagers, the relative angle, and in some cases also the relative position, between the images produced by these real-time imagers and the optical detector attached thereto should be determined prior to use.

System 500 is used for tracking first medical tool 514 relative to second medical tool 516 and present on display 512 information related to both tools, at the correct spatial relationship there between. For example, when first medical tool 514 is a needle and second medical tool 516 is an ultrasound imager, ultrasound imager 516 acquires a real time image or images of a region of interest of the body of patient 520 (e.g., the abdomen, the embryo). A physician 522 inserts needle 514 toward the region of interest.

WFOV optical detector 502 and optical detector 504 acquire an image or images of the light emitters within the FOV thereof. In Figure 13, WFOV optical detector 502 acquires an image or images of light emitters $508_1$ and $508_2$ and optical detector acquire 504 acquire an image of light emitter 506. Processor 510 determines the position and orientation of WFOV optical detector 502 relative to optical detector 504, and consequently in reference coordinate system 518, according to the representations of light emitters $508_1$ and $508_2$ and the representation of light emitter 506 (i.e., as determined from the acquired images either by WFOV optical detector 502 and optical detector 504 respectively or by processor 510). Thus processor 510 determines the position and orientation of the needle 514 relative to ultrasound imager 516. Display 512, displays the acquired ultrasound image along with a representation of needle 514 and optionally with a representation of the projected path of needle 514 in the region of interest, superimposed on the acquired image.

It is noted that an additional optical detector, which may be a WFOV optical detector, and an additional light emitter may both be located, for example of the head of physician 522. Processor 510 then determines the relative position and orientation between the head of the physician and medical tool 516. Thus, when medical tool 516 is for example an ultrasound imager, processor 510 may render the ultrasound image such that display 512 displays the ultrasound image at the orientation corresponding to the point of view of physician 522

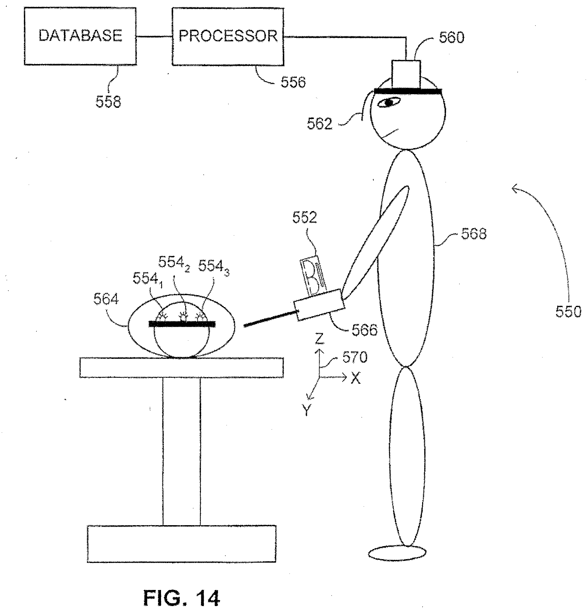

Reference is now made to Figure 14, which is a schematic illustration of an exemplary WFOV medical tracking system, generally reference 550 in accordance with another embodiment of the disclosed technique. System 550 includes a WFOV optical detector 552, at least three light emitters $554_1$, $554_2$ and $554_3$, a processor 556, a database 558 and a display such as Head Mounted Display (HMD) 560. HMD 560 includes a visor 562. Alternatively HMD 314 may be in the form of near-eye-display.

Processor 556 is coupled with database 558, WFOV optical detector 552, HMD 560. When light emitters $554_1$, $554_2$ and $554_3$ are LEDs, processor 556 is optionally coupled therewith. Light emitters $554_1$, $554_2$ and $554_3$ are attached to a body location of patient 564 and WFOV optical detector is attached to a medical tool 566 held by a physician 568. System 550 is associated with a reference coordinate system 570, which in this case is also the coordinate system associated with patient 564.

System 550 may be employed for tracking medical tool 566 relative to patient 564. System 550 may also be employed for presenting data acquired by medical tool 566 at the location from which that data was acquired. To that end WFOV optical detector 552 acquires an image or images of light emitters $554_1$, $554_2$ and $554_3$ within the FOV thereof. Processor 566 determines the relative position and orientation between WFOV optical detector 552 (i.e., the tool) and the patient according to the representations of light emitters $554_1$, $554_2$ and $554_3$ (i.e., as determined either by WFOV optical detector 552 or by processor 566). Furthermore, processor 556 may further constructs a visual representation (not shown) of the medical tool 566 or at least of the obscured portion of thereof. Medical tool 566, along with the visual representation thereof, is presented by HMD 560 on visor 562 to physician 568. Accordingly, physician 568 can view the representation of medical tool 566 superimposed on a model of patient 564. When medical tool 566 is an ultrasound imager, the image produced by the ultrasound imager may be displayed to physician 568 superimposed on a model of patient 564 corresponding to the location in the body of patient 564 at which the image was acquired. When medical tool 566 is a four dimensional (4D) ultrasound transducer (i.e., acquiring a live 3D image), the live 3D ultrasound image may be displayed. Additionally, a 3D ultrasound model can be built based upon regular 2D ultrasound images, each taken at different positions and orientations in the coordinate system associated with patient 564.

As mentioned above, the display employed in any of the above WFOV medical tracking systems may a hand held display which includes a camera. The relative position and orientation between the hand held display and the patient can be tracked (e.g., the hand held device is one of the target objects). Furthermore, the hand held device acquires an image of the patient. Accordingly, the above mentioned model of the patient may be superimposed on the image acquired by the hand held device. It is noted that the systems described above in conjunction with Figures 9, 10, 11, 12, 13 and 14 are brought herein as examples only. The configuration of the detectors and light emitters may change according to specific needs. For example, all the optical detectors may be WFOV optical detectors. Alternatively, in Figures 10, 11 and 12 a WFOV optical detector may be located on the head of the physician and optical detectors on the patient and medical tool. In Figure 9, the locations of the WFOV optical detector and the optical detector may be interchanged. Furthermore, in conjunction with Figures 9, 10, 11 and 12 the WFOV optical detector may be fitted with two light emitters and the optical detector with one light emitter. Additionally, more than three light emitters may be associated with each pair of reference location and target object, thus increasing the accuracy and reliability of the tracking system.

As mentioned above in conjunction with Figure 12, the system according to the disclosed technique, may include two or more WFOV optical detectors attached to any one of the HMD or the target object, thus further increasing the FOV of tracking system. When two or more WFOV optical detectors are employed (e.g., on medical tool 472 - Figure 12, Medical tool 418 - Figure 11), each WFOV detector is associated with a respective light emitter. One of these WFOV optical detectors is selected to be an active WFOV optical detector, which acquires an image or images of the light emitters within the FOV thereof, similar as explained above. The processor (e.g., processor 464 - Figure 12, Processor 410 - Figure 11, Processor 360 - Figure 10) selects the active WFOV optical detector, for example, by tracking the representations of the light emitters in the image acquired by the optical sensor of the active WFOV optical detector. For example, when these representations approach the edge of the sensor of the currently active WFOV optical detector (e.g., within a determined threshold), then, an adjacent WFOV optical detector is selected as the active WFOV optical detector. According to another example, when the representations of the light emitters in the image acquired by the optical sensor of the active WFOV optical detector disappear, for example, due to an obstruction in the FOV of the active WFOV optical detector or due to high intensity ambient light (e.g., caused by the sun or operating room lights), another WFOV optical detector is selected as the active WFOV optical detector. When the system starts-up, the processor may toggle the active sensor until the light emitters are detected in one of the sensors, and from there on it controls the sensors as described above and below.

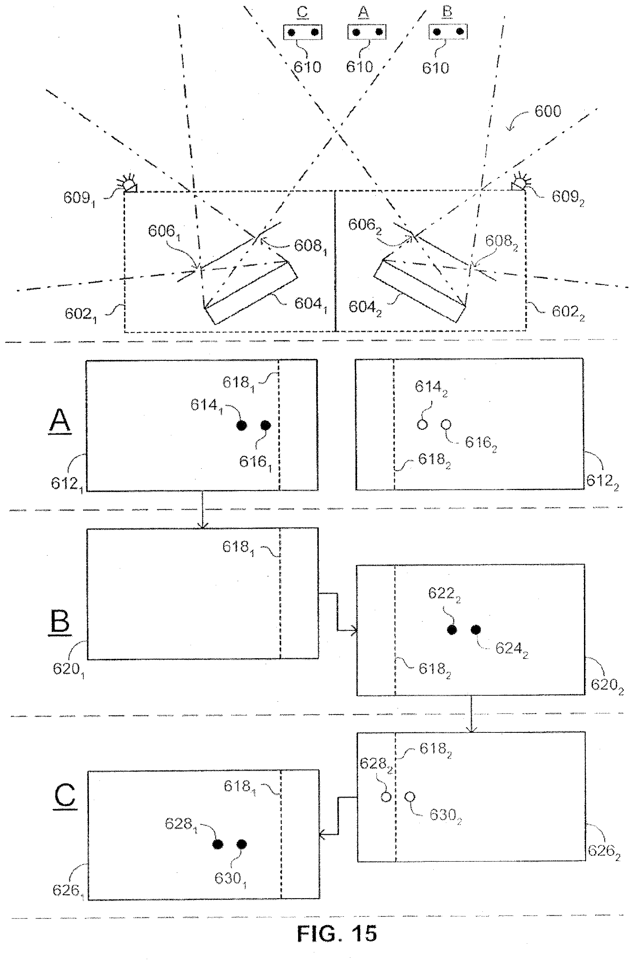

It is noted that in practice, the FOV respective of each of the WFOV optical detectors should partially overlap. It is further noted that in practice, the WFOV optical detectors may include the logic for selecting the active WFOV detector rather the processor, for example, based on the above mentioned BLOB analysis which provides information relating to the location of the representations of the light emitters on the optical sensor. Reference is now made to Figure 15, which is a schematic illustration of an optical detector assembly, generally referenced 600 and the operation thereof, in accordance with a further embodiment of the disclosed technique. Optical detector assembly 600 includes two WFOV optical detectors $602_1$ and $602_2$. WFOV optical detector $602_1$ includes a respective sensor $604_1$ and respective optical receptors $606_1$ and $608_1$. WFOV optical detector $602_2$ includes a respective sensor $604_2$ and respective optical receptors $606_2$ and $608_2$. Optical receptors $606_1$ and $608_1$ are optically coupled with sensor $604_1$. Optical receptors $606_2$ and $608_2$ are optically coupled with sensor $604_2$. Each WFOV optical detector $602_1$ and $602_2$ is associated with a respective one of light emitters $609_1$ and $609_2$. Also, WFOV optical detectors $602_1$ and $602_2$ are tilted one with respect to the other. Light emitters $609_1$ and $609_2$ may also be tilted one with respect to the other.

Figure 15 depicts a light emitters assembly 610, which includes two light emitters, located, for example, on an HMD (e.g., Light Emitters $456_1$ and $456_2$ located on HMD 468 - Figure 12). Light emitters assembly moves relative to optical detector assembly 600 (i.e., either light emitters assembly 610 moves or optical detector assembly 600 rotates or both), through three successive different positions marked 'A', 'B' and 'C'. When light emitters assembly 610 is in position 'A', WFOV optical detector $602_1$ is the active WFOV optical detector. The image $612_1$ acquired by optical sensor $604_1$ includes two representations $614_1$ and $616_1$, each associated with a respective one of the light emitters in light emitters assembly 610. When light emitters assembly 610 is in position 'A', WFOV optical detector $602_2$ is inactive and does not acquire an image. However, image $612_2$ is brought herein to illustrate that light emitted by light emitters assembly 610 may still impinge on optical sensor $604_2$ as indicated by hollow circles $614_2$ and $616_2$.

As light emitters assembly 610 moves toward position 'B', the location of representations $614_1$ and $616_1$ is tracked to determine if the location of at least one of representations $614_1$ and $616_1$ pass threshold $618_1$ in the direction in which sensor $604_2$ is located. Threshold $618_1$ is a line of pixels on sensor $604_1$. When one of representations $614_1$ and $616_1$ pass threshold $618_1$, WFOV optical detector $602_2$ is selected as the active WFOV optical detector. The image $620_2$ acquired by optical sensor $604_2$ includes two representations $622_2$ and $624_2$, each associated with a respective one of the light emitters in light emitters assembly 610. When light emitter 610 is in position 'B', WFOV optical detector $602_1$ is inactive and does not acquire an image. Furthermore, since light emitters assembly 610 is not within the FOV of first WFOV optical detector $602_1$, no light impinges thereon, as illustrated in image $620_1$. It is noted that image $620_1$ is brought herein for the purpose of illustration only. Such an image is not actually acquired.

After moving to Position 'B', light emitters assembly 610 moves toward position 'C'. The location of representations $622_2$ and $624_2$ is tracked to determine if the location of at least one of representations $622_2$ and $624_2$ pass threshold $618_2$ in the direction in which sensor $604_1$ is located. When representation $622_2$ passes threshold $618_2$, WFOV optical detector $602_1$ is selected as the active WFOV optical detector. The image $626_1$ acquired by optical sensor $604_1$ includes two representations $628_1$ and $630_1$, each associated with a respective one of the light emitters in light emitters assembly 610. When light emitter 610 is in position 'C', WFOV optical detector $602_2$ is inactive and does not acquire an image. However, image $626_2$ is brought herein to illustrate that light emitted by light emitter 610 may still impinge on optical sensor $604_2$ as indicated by hollow circles $628_2$ and $630_2$.

In general, only the light emitter associated with active WFOV optical detector is employed for determining the position and orientation of the target object. When light emitters $609_1$ and $609_2$ are LEDs, only the LED (or LEDs) associated with the active WFOV optical detector is activated (i.e., the operation of the LEDs is synchronized according to the currently active WFOV optical detector). With reference to Figure 15, when light emitters $609_1$ and $609_2$ are LEDs, in position 'A' WFOV optical detector $602_1$ is the active WFOV optical detector and light emitter $609_1$ is activated. In position 'B' WFOV optical detector $602_2$ is the active WFOV optical detector and light emitter $609_2$ is activated. In position 'C' WFOV optical detector $602_1$ is again the active WFOV optical detector and light emitter $609_1$ is activated. When the light emitters associated with the two or more WFOV optical detectors are light reflectors, then the representation or representations of the light emitter associated with the active WFOV optical detector may be determined according to the intensity of the representations. For example, the representation of the light emitter associated with the active WFOV optical detector shall exhibit a higher intensity relative to the light emitter associate with the inactive WFOV optical detector. As another example, the size of the representation of the light emitter associate with the active WFOV optical detector shall be larger than the size of the other light emitter. Furthermore, when the spatial relationship between the two light emitters is known, the representations of both may be employed for determining position and orientation. Nevertheless, a single light emitter, attached to said target object may also be employed for determining the position and orientation of the target object. Thus, this single light emitter is associated with both WFOV optical detectors $602_1$ and $602_2$. The angular span of the light emitted by the single light emitter should be similar to the FOV spanned by both WFOV optical detectors $602_1$ and $602_2$.

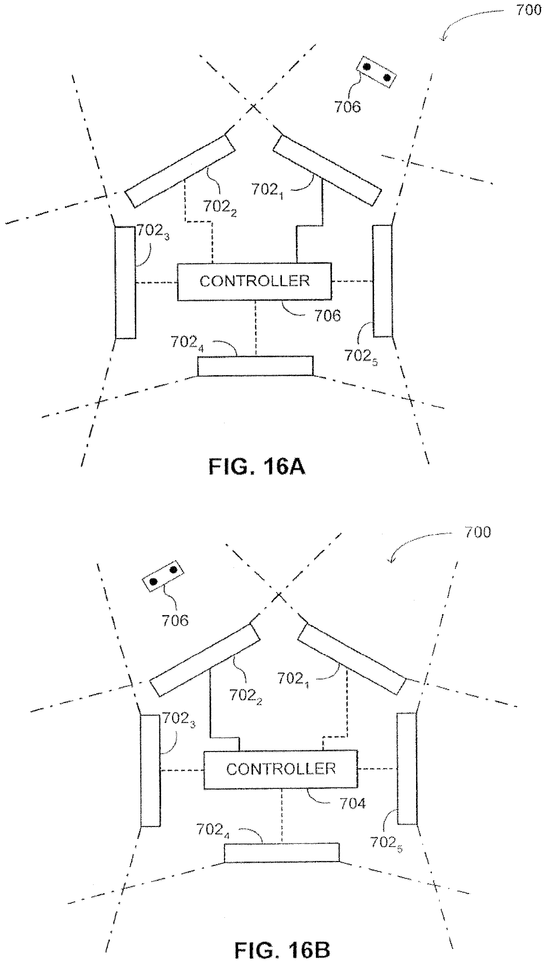

The number of optical detectors in an optical detector assembly is not limited to two. More than two optical sensors may be employed where one is selected as the active sensor. Reference is now made to Figures 16A-16E, which are schematic illustrations of an optical detector assembly, generally referenced 700 and the operation thereof, in accordance with another embodiment of the disclosed technique. Optical detector assembly 700 includes a plurality of optical sensors. In Figures 16A-16E, optical detector assembly 700 is exemplified as including five optical detectors $702_1$, $702_2$, $702_3$, $702_4$ and $702_5$ and a controller 704. Each one of optical detectors $702_1$, $702_2$, $702_3$, $702_4$ and $702_5$ is configured to be coupled with controller 704. Each one of optical detectors $702_1$, $702_2$, $702_3$, $702_4$ and $702_5$ is associated with a respective light emitter (not shown) and further include a sensor and an entrance pupil (also not shown). As mentioned above, optical detector assembly 700 may include a single light emitter, where the angular span of this single light emitter similar to the FOV spanned by optical detectors $702_1$, $702_2$, $702_3$, $702_4$ and $702_5$ (e.g., a reflective sphere). Thus, this single light emitter is associated with all of optical detectors $702_1$, $702_2$, $702_3$, $702_4$ and $702_5$. Furthermore, the number of light emitter may be larger than one but smaller than the number of optical detectors (e.g., 2 light emitters and 5 optical detectors). In such a configuration, even when one of the light emitters is obscured (e.g., either from the optical detector on the HMD or, when the light emitter is a light reflector from the light source illuminating this light reflector), the other light emitter one may be employed to determine the position and orientation of the target object.

Optical detector assembly 700, is located for example on a patient or a medical tool as described above, and acquires images of light emitters assembly 706, located, for example, on an HMD. Optical detector assembly 700 and light emitters assembly 706 may exhibit relative motion (i.e., translation and rotation) therebetween. Similar to as explained above in conjunction with Figure 15, controller 704 may be embedded within a processor (e.g., processor 464 - Figure 12) or within the optical detector assembly which may include the logic for selecting the active sensor. When the logic for selecting the active detector is executed in a processor, the optical detector assembly may include a component for communicating with the processor and for activating and de-activating the sensors according to that logic.

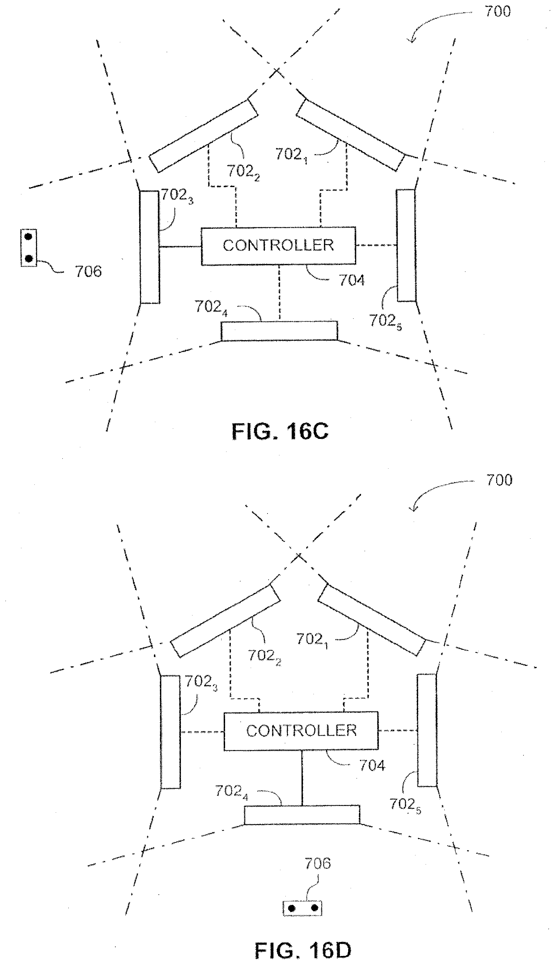

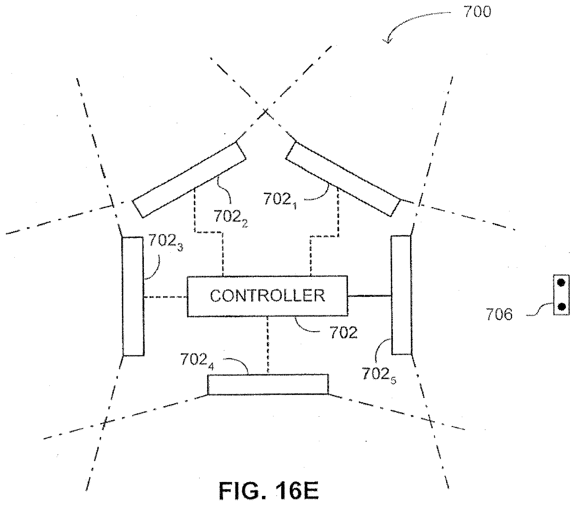

In Figure 16A, light emitter assembly 706 is located within the FOV of sensor $702_1$. Thus, controller 704 selects optical detector $702_1$ as the active sensor, as indicated by the solid line (i.e., as opposed to the dashed lines between controller 704 and optical detectors $702_2$, $702_3$, $702_4$ and $702_5$). The position and orientation of the target object is determined according to an image acquired by optical detector $702_1$. In Figure 16B, light emitter assembly 706 moved into the FOV of sensor $702_2$. Thus, controller 704 selects optical detector $702_2$ as the active sensor, as indicated by the solid line (i.e., as opposed to the dashed lines between controller 704 and optical detectors $702_1$, $702_3$, $702_4$ and $702_5$). The position and orientation of the target object is determined according to an image acquired by optical detector $702_2$. In Figure 16C, light emitter assembly 706 moved into the FOV of sensor $702_3$. Thus, controller 704 selects optical detector $702_3$ as the active sensor, as indicated by the solid line (i.e., as opposed to the dashed lines between controller 704 and optical detectors $702_1$, $702_2$, $702_4$ and $702_5$). The position and orientation of the target object is determined according to an image acquired by optical detector $702_3$. In Figure 16D, light emitter assembly 706 moved into the FOV of sensor $702_4$. Thus, controller 704 selects optical detector $702_4$ as the active sensor, as indicated by the solid line (i.e., as opposed to the dashed lines between controller 704 and optical detectors $702_1$, $702_2$, $702_3$ and $702_5$). The position and orientation of the target object is determined according to an image acquired by optical detector $702_4$. In Figure 16E, light emitter assembly 706 moved into the FOV of sensor $702_5$. Thus, controller 704 selects optical detector $702_5$ as the active sensor, as indicated by the solid line (i.e., as opposed to the dashed lines between controller 704 and optical detectors $702_1$, $702_2$, $702_3$ and $702_4$). The position and orientation of the target object is determined according to an image acquired by optical detector $702_5$.

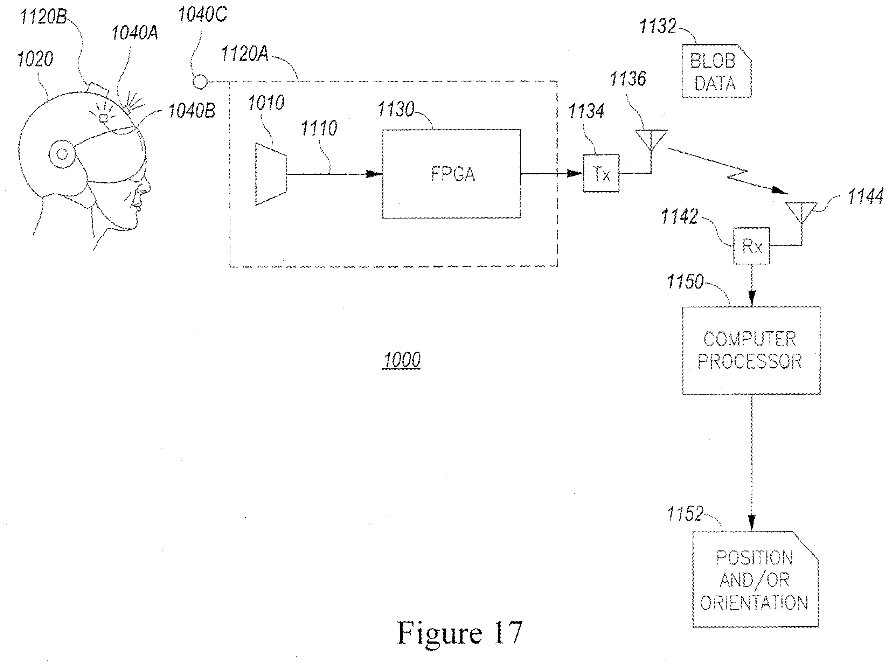

The description which follows, including figures 17-21, provides examples not forming part of the invention but which are useful for understanding the invention. Tracking objects using video cameras requires both bandwidth and computational power that inevitably lead to latency issues. In some applications it is desirable to be able to track objects quickly without the need for high computational power or a high bandwidth of the communication link between the tracking device and the control module. According to some additional embodiments of the disclosed technique, the tracker may include at least two optical tracker sensors (i.e., optical detectors), facing at least partially each other (i.e., within the FOV of each other). Each optical tracker sensor may include: at least one pixel array sensor (i.e., an optical sensor) configured to generate a stream of pixel values representing a scene; at least one visual indicator (i.e., a light emitter) physically coupled to said at least one pixel array sensor; and an integrated circuit (IC) physically coupled to said at least one pixel array sensor, and configured to: receive said stream of pixel values; and apply a binary large object (BLOB) analysis to said stream, to yield BLOB parameters indicative of the at least one visual indicator present in the scene in a single pass of the pixels representing the scene; and a computer processor configured to receive said BLOB parameters and calculate a relative position and/or orientation, or a partial data thereof, of the at least two optical tracker sensors.

Figure 17 is a diagram illustrating a system according to embodiments of the disclosed technique. An optical tracker 1000 is illustrated and may include: at least two optical tracker sensors such as sensor 1120A which includes at least one pixel array sensor 1010 configured to generate a stream of pixel values representing a scene containing a plurality of visual indicators such as 1040A and 1040B affixed to an object 1020 (such as a helmet) on which another optical sensor 1120B is located facing optical tracker sensor 1120A which is also coupled with a visual indicator 1040C. Optical tracker sensor 1120A may further include an integrated circuit (IC) such as a field programmable gate array (FPGA) 1130 which may also be implemented as an application specific integrated circuit (ASIC) physically coupled to said pixel array sensor 1010 possibly by interface 1110. It is noted that the system may be implemented by any combination of hardware and software as may be suitable and desirable as per the specific application. It is further understood that a single IC may be in communication with a plurality of pixel array sensors and so the single IC may apply the BLOB analysis to data coming from any of the plurality of the pixel array sensors.

In operation, IC 1130 may be configured to receive the aforementioned stream of pixel values and apply a single pass binary large object (BLOB) analysis to said stream, to yield BLOB parameters 1132 indicative of the at least one visual indicator. Optical tracker 1000 may further include a computer processor 1150 configured to receive said BLOB parameters 1132 optical tracker sensors 1120A and 1120B and calculate at least one of: a position, an orientation, or partial data thereof 1152 of optical tracker sensor 1120B relative to said optical tracker sensor 1120A. Architecturally, in one embodiment, computer processor 1150 may be packed within said optical tracker sensor 1120A in order to provide compactness and ease of use.

The aforementioned configuration, namely of two optical tracker sensors 1120A and 1120B facing each other, at least one visual indicator 1040C coupled to one of the optical tracker sensors 1120A, and at least two visual indicators 1040A and 1040A coupled to the other optical tracker sensor 1120B, is sufficient for calculating the full six degrees of freedom of the relative position and orientation between the two optical tracker sensors.

A significant advantage of this configuration over currently available optical tracking configurations is that this configuration supports a compact design of at least one of the optical tracker components, namely the optical tracker sensor which is coupled with the single visual indicator. Conversely, in currently available optical tracker configurations, where a single camera faces at least three visual indicators coupled to an object (three visual indicators are the minimal number of visual indicators required for full position and orientation), the distance between any of two visual indicators is required to be greater than a minimal distance which is proportional to the distance between the optical tracker sensor and the tracked object, and further proportional to the required accuracies of the tracker (i.e., better accuracy requires bigger distance). The minimal distance can be reduced if two optical tracker sensors which are separated by yet another minimal distance are used to track the object. In the aforementioned configuration, the single visual indicator and the optical tracker sensor which are coupled to each other can be encapsulated within a compact housing, wherein the size of the housing is almost as small as desired. Namely, the size is limited only by the size of the hardware components it comprises and not by any mathematical or physical limitations that stem from the required accuracies and the distance between this optical tracker component and other components in the system.

The compact design in accordance with embodiments of the disclosed technique is specifically advantageous when the compact component is attached to a hand-held object or to a head-mounted unit as will be detailed below. Other advantages arise from the accuracy and the short and long term mechanical stability of the relative position between the components comprising the optical tracker unit (i.e. the sensor and visual indicator) which itself is required for system accuracy. For example, when the visual indicator is a light emitting diode (LED), such that the LED and the pixel array sensor may both be assembled to a single printed circuit board (PCB), their relative position can be known with the very high accuracies promised by the PCB assembly line. This configuration is also robust to changes due to environmental conditions (e.g. temperature) and mechanical strains. However, generally the wire used to transmit the video from the sensor to the processor is required to support a large bandwidth in order to support a short transmission time of every video frame and thus keep the system latency small. Moreover, sometimes the application requires that no cable is used.

The disclosed technique, in embodiments thereof, addresses the aforementioned challenges of the currently available optical tracker. As explained above, using BLOB analysis reduces the amount of data that need to be transmitted for processing. Yet another embodiment for compact low latency optical tracker may use a video imaging device which is configured to extract pixels only in a predefined region of interest (ROI) instead of the aforementioned BLOB based optical tracker sensor. The video capturing device (which replaces in this embodiment optical sensor 1120A and/or 1120B) is configured to capture only the ROI which is set to contain the range in which the visual indicators coupled to the other sensor. More variation of the ROI will be explained below. Both BLOB and ROI solutions support a low bandwidth wired or wireless configuration and both embodiments can be used in a single implementation.

Another advantage of the compact design is the possibility to couple several pairs of pixel array sensors and LEDs in a single optical tracker sensor, such that each pair covers a different FOV, and thus a single optical tracker sensor may cover a very large FOV and still remain compact. Only a single pair is required to be employed at any given tracker cycle and therefore the distance between the pairs can be kept minimal.

Determining the BLOB parameters may be achieved by methods of single-pass BLOB analysis known in the art. The single-pass BLOB analysis, as described herein, relates to the ability to scan an entire image and detect all of the objects in the scene and derive their respective BLOB parameters in a single pass of the pixels (as opposed to two and three passes that were required in previous techniques).

In one embodiment of the disclosed technique, a threshold approach is being implemented. Accordingly, each pixel is considered as belonging to a BLOB if its gray-level value is higher than a predetermined threshold. This approach works best in cases where the image contains a relatively small number of BLOBs in a dark background, which is usually the case in optical tracker applications, where various optical and electro-optical means are used in order to render visual indicators more distinguishable over their surroundings.

In one embodiment, the pixels coming from the sensor are read one by one. Once a current pixel is determined as being above a predefined threshold, its BLOB-associated neighboring pixels (e.g., located one or two pixels apart) are also being checked. The current pixel is being associated with the BLOB with which the neighboring pixels are associated. In a case that two neighboring pixels are associated with two different BLOBs, an indication for uniting the two BLOBs is being made. Other manners to implement the BLOB detection are possible. It is understood that other embodiments such as reading the pixels two by two or any other number may also be contemplated.

According to some embodiments of the disclosed technique, computer processor 1140 and the at least one optical tracker sensor 1120 may be located separately and wherein the optical tracker sensor is further configured to transmit the BLOB parameters 1132 via transmitter 1134 and antenna 1136 to antenna 1144 and receiver 1142 coupled to computer processor 1140. One of the advantages of the disclosed technique is that since the BLOB analysis is carried out in situ (i.e., at the capturing device), the transmitted parameters are not raw data such as video sequence (such as the case with video trackers) and therefore do not require a broad bandwidth. Another advantage of the disclosed technique is that since the BLOB analysis is performed on a single-pass basis and only the BLOB parameter are transmitted, any unnecessary latency between the time when an image is sampled by the pixel array sensor 1010 and the time when the computer processer 1140 starts its calculation is eliminated.

According to some embodiments of the disclosed technique, where one of the at least two optical tracker sensors is coupled to two visual indicators or more, the second optical tracker sensor facing it only requires a single visual indicator. This requirement stems from the fact that for deriving full relative position and orientation data (i.e., six degrees of freedom) for a pair of optical tracker sensors, at least three visual indicators are required in total.

This configuration may advantageously enable designing a very compact single-visual-indicator optical tracker sensor. The compact optical tracker sensor may be used for coupling to objects to-be-tracked, where small size is an essential advantage such as hand-held devices. The compact optical tracker sensor may include a housing, encapsulating both the single visual indicator and the pixel array sensor. In a non-limiting example, the single visual indicator may be located at a distance less than approximately 5cm from a center of the pixel array sensor.

According to some embodiments of the disclosed technique, visual indicator 40 may consist of at least one of: a light source, and a reflector or a combination thereof. In addition, the said light source or the light source emitting the light reflected by the reflector may be configured to emit light pulses and wherein the optical tracker further comprising means for synchronization between the pulses of the light source and the integration time of the pixel array sensor 1010.

According to some embodiments of the disclosed technique, the stream of pixels is only transferred for at least one predefined region of interest (ROI) within the pixel array sensor 1010, where the ROI borders are updated on a frame-by-frame basis. This will further reduce the time elapsed between the capturing of the scene and the completion of the BLOB analysis, since the readout time of the pixels from the sensor 1010 will be shorter. This approach works best in cases where the image contains a relatively small number of BLOBs that are restricted to a small part of the image, and their expected position can be roughly predicted based on the past, as is the case when tracking a single object having one or more visual indicators attached to it.

Determining the ROI borders may be carried out based on the predicted locations of the objects to be tracked. Additionally, whenever the sensor technology allows, two or more ROIs may be generated in order to track two or more groups of visual indicators with minimal latency. For example, whenever two objects, each having visual indicators need to be tracked. Alternatively, each object can be independently and separately tracked by synchronizing the periods of time in which the different sensors and light sources are operated.

According to other embodiments, a complementary technology such as magnetic or inertial tracking may be used in case of a temporary loss of optical detection of the visual indicators. Upon resuming of the optical detection, the optical tracker may use the ROI derived from the complimentary tracker (magnetic, inertial, or other). Alternatively, after long periods of loss of optical detection, the optical tracking is resumed in full frame (non-ROI mode).

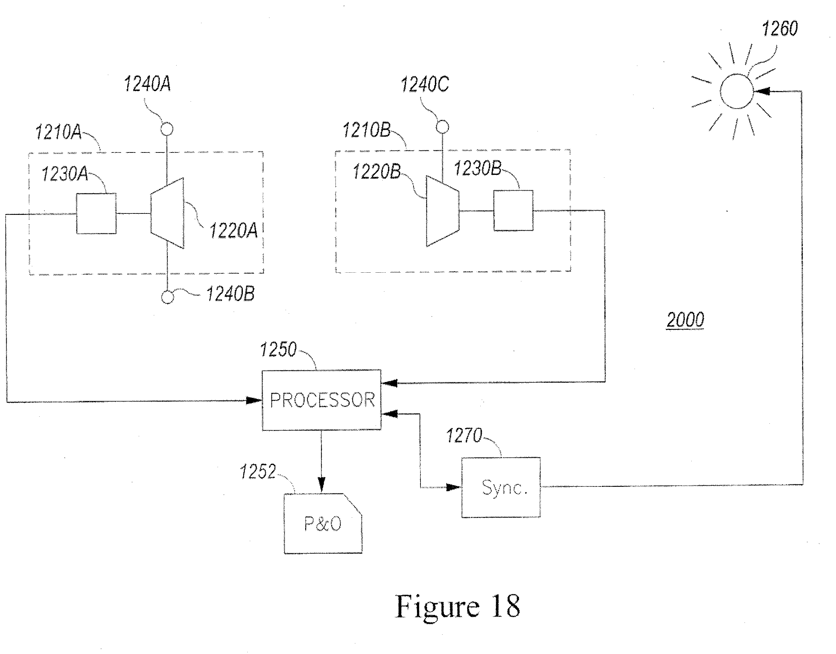

Figure 18 is a diagram illustrating another aspect of a system according to embodiments of the disclosed technique. Optical tracker 2000 may include: at least two optical tracker sensors 1210A and 1210B facing at least partially each other wherein at least one of said optical tracker sensors includes at least one pixel array sensor 1220A and 1220B configured to generate a stream of pixel values representing a scene; an integrated circuit (IC) 1230A and 1230B physically coupled to said at least one pixel array sensor 1220A and 1220B, and configured to: receive said pixel-by-pixel stream of values; and apply a binary large object (BLOB) analysis to said stream, to yield BLOB parameters indicative of the at least one visual indicator 1240A, 1240B or 1240C present in the scene in a single pass of the pixels representing the scene.

Optical tracker 2000 may also include at least one visual indicator 1240A, 1240B or 1240C coupled to each of the at least two optical tracker sensors 1210A and 1210B. Optical tracker 2000 may also include a computer processor 1250 configured to receive said BLOB parameters and calculate a relative position and/or orientation 1252, or a partial data thereof, of the at least two optical tracker sensors.

According to some embodiments of the disclosed technique, the total number of the visual indicators physically attached to the at least two optical tracker sensors is at least three.

According to some embodiments of the disclosed technique, at least one of the at least two optical tracker sensors is an image capturing device, and wherein the computer processor is configured to calculate the relative position and/or orientation, or a partial data thereof, further based on data derived from the image capturing device.

According to some embodiments of the disclosed technique, at least one of the at least two optical tracker sensors is stationary.

According to some embodiments of the disclosed technique, the computer processor is packed within one of said optical tracker sensors.

According to some embodiments of the disclosed technique, the computer processor and at least one of the optical tracker sensors are located separately from each other and wherein the at least one optical tracker sensor is further configured to transmit the BLOB parameters over a wired communication channel to the computer processor.

According to some embodiments of the disclosed technique, the computer processor and at least one of the optical tracker sensors are located separately from each other and wherein the at least one optical tracker sensor is further configured to transmit the BLOB parameters over a wireless communication channel to the computer processor.

According to some embodiments of the disclosed technique, the at least one visual indicator comprises at least one of: a light source, and a reflector.

According to some embodiments of the disclosed technique, at least one visual indicator is configured to emit or reflect light pulses and wherein the optical tracker further comprises means for synchronization 1270 between the light pulses from light source 1260 or the visual indicators 1240A-C and the at least two optical tracker sensors.

According to some embodiments of the disclosed technique, the optical tracker is operable in a region of interest (ROI) mode in which only a subset of the stream of pixels is transferred to the IC, wherein the subset of stream of pixels represents a predefined subset of the pixel array associated with the ROI.

According to some embodiments of the disclosed technique, the ROI is determined on a frame-by-frame basis based on predicted locations of the at least one visual indicator.

According to some embodiments of the disclosed technique, the optical tracker further comprises magnetic or inertial or other tracking means configured to provide tracking data whenever the optical tracker sensors fail, and wherein the optical tracker is configured to resume optical tracking with an ROI that is determined based on the data provided by the magnetic or inertial or other tracking means.

According to a preferred embodiment of the disclosed technique, the optical tracker comprises two optical tracker sensors and the total number of the visual indicators physically attached to the two optical tracker sensors is at least three. This way, full position and orientation representation may be derived. It is understood however, that using less visual indicators may provide partial position and orientation data that may be beneficial for some applications.

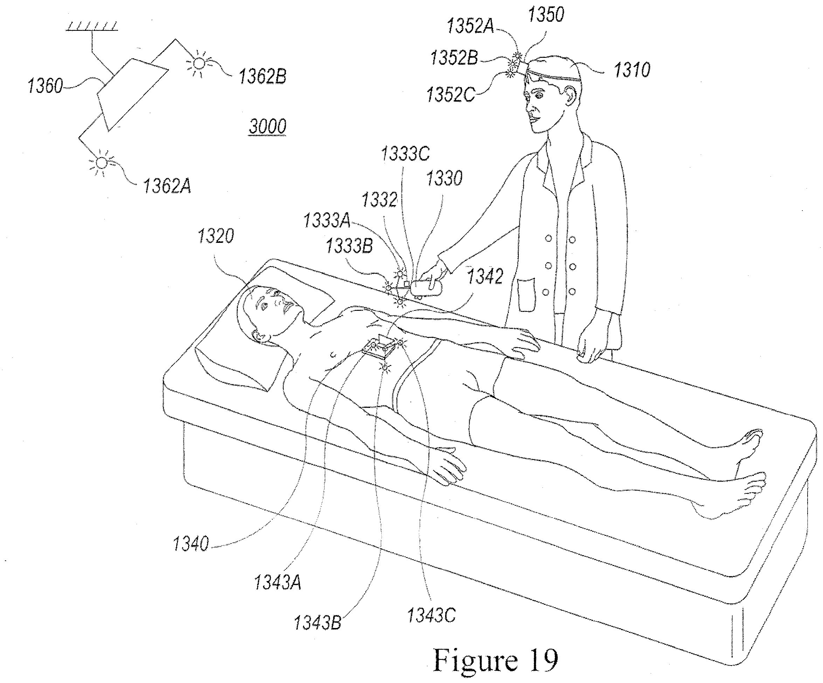

Figure 19 is a diagram illustrating non-limiting exemplary applications of the system according to embodiments of the disclosed technique. Environment 3000 illustrates an operating room in which a doctor 1310 is using a hand held medical device 1330 to which the optical sensor 1332 and visual indicators 1333A, 1333B, and 1333C are attached. Similarly, optical sensor 1350 and visual indicators 1353A, 1353B, and 1353C may be mounted on the head of the doctor 1310 possibly with a head mounted display system (not shown). Another optical tracker sensor 1360 may be stationary (e.g., attached to the ceiling) and may include at least two visual indicators 1362A and 1362B. Yet another optical sensor 1342 and visual indicators 1343A, 1343B, and 1343C may be patient-mounted to a fixture 1340 on a patient 1320.

According to some embodiments, the hand-held device 1330 may be any operating tool (e.g., a scalpel, a laparoscope tube, an ultrasound transducer or a needle). In this manner, the hand held device may be tracked. It is understood that since the objects to be tracked and the optical tracker sensors coupled thereto define a specific spatial relationship, in order to calculate the relative position and orientation of two objects (as opposed to the relative position and orientation of two optical tracker sensors) in reality, the aforementioned specified spatial relationship needs to be known. There are several ways known in the art for calibration or registration of that spatial relationship.

When the optical tracker sensor 1342 is patient-mounted, e.g. physically attached to the patient's head or limb, the fixture 1340 on the patient may be tracked. This attachment may be direct or indirect, e.g. the optical tracker sensor may be attached directly to the head of the patient, or may be attached to a frame which is rigidly attached to the head of the patient.

According to some embodiments of the disclosed technique, head-mounted optical sensor or hand-held device optical sensor or patient-mounted optical sensor is located within a field of view of at least one of the at least two optical tracker sensors, and wherein the computer processor is further configured to calculate a position and/or orientation, or a partial data thereof, of the head-mounted optical sensor or hand-held device optical sensor or patient-mounted optical sensor, relative to at least one of the at least two optical tracker sensors.

According to some embodiments of the disclosed technique, at least one of the two optical tracker sensors is patient-mounted or physically attached to a hand-held device and having at least one visual indicator attached thereto, and wherein the head-mounted optical tracker sensor has at least two visual indicators attached thereto.

It is understood that many more combinations and variations of the aforementioned optical sensors and visual indicators may be used, as required per the use or the design of the optical tracking system.

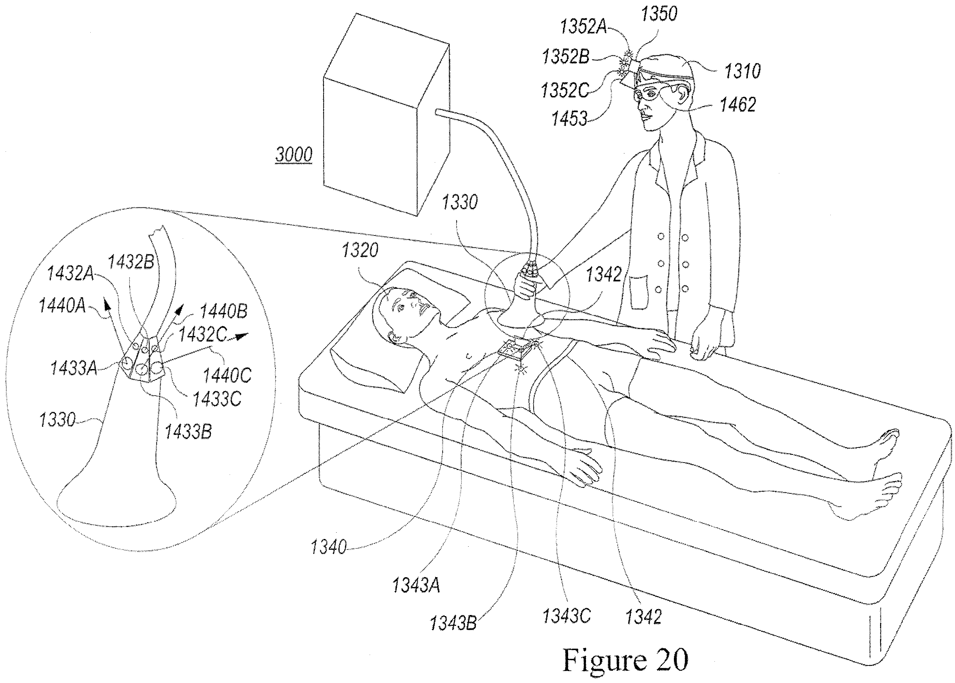

Figure 20 is a diagram illustrating yet another non-limiting exemplary application of the system according to embodiments of the disclosed technique. Environment 3000 illustrates an operating room in which a human user 1310 is donning a head mounted display system which also comprises an optical tracker sensor 1453 and at least two visual indicators 1352A-1352C. Additionally, Human user 1310 may be using a hand held medical device 1330 to which an optical tracker sensor comprising a plurality of pixel array sensors 1433A-1433C and a single IC (not shown) is attached. Pixel array sensors 1433A-1433C are positioned along the perimeter of hand held medical device 1330 wherein each one of pixel array sensor 1433A-1433C is radially facing a different sector which may or may not be adjacently overlapping. Preferably but not exclusively, the pixel array sensors 1433A-1433C may each be tilted so that the entire structure with the bases of the sensors form a pyramid.

Similarly, corresponding visual indicators such as 1432A-1432C, preferably light sources such as LEDs are also located along the perimeter of hand held medical device 1330 and radially facing different sectors so as to be located in proximity to the respective pixel array sensors 1433A-1433C. Preferably, but not exclusively, the distance between a pixel array sensor of 1433A-1433C and its corresponding light source of 1432A-1432C does not exceed 3 centimeters and the distance between the pixel array sensors 1433A-1433C does not exceed 5 centimeters. These exemplary limitations enable a compact arrangement of the pixel array sensors and the light sources on a hand held device. The tilt angle provides ergonomic advantages, specifically if the head of the human user 1310 is facing down where the hand held device 1330 is positioned.

In operation, a dynamic selection of which one of pixel array sensors 1433A-1433C should be employed in every given tracker cycle is being carried out. Similarly, the corresponding light source (being the one proximal to the pixel array sensor) is also employed. Head mounted optical tracker sensor 1453 is constantly capturing one of the light sources 1432A-1432C which are being selectively operated based on the spatial angle of the hand held device. In the same time only one of pixel array sensor 1433A-1433C whose field of view (FOV) includes the visual indicators 1352A-1352C of the head mounted device 1350 is being employed at a given cycle.

The aforementioned design combines the benefits of robust tracking with compactness of the tracker. The logic enables a more robust tracking as the hand held device is not limited by a narrow field of view of a single optical sensor. The aforementioned structure provides compactness which is specifically advantageous where the optical sensors need to be attached to a hand held device where compactness is crucial.

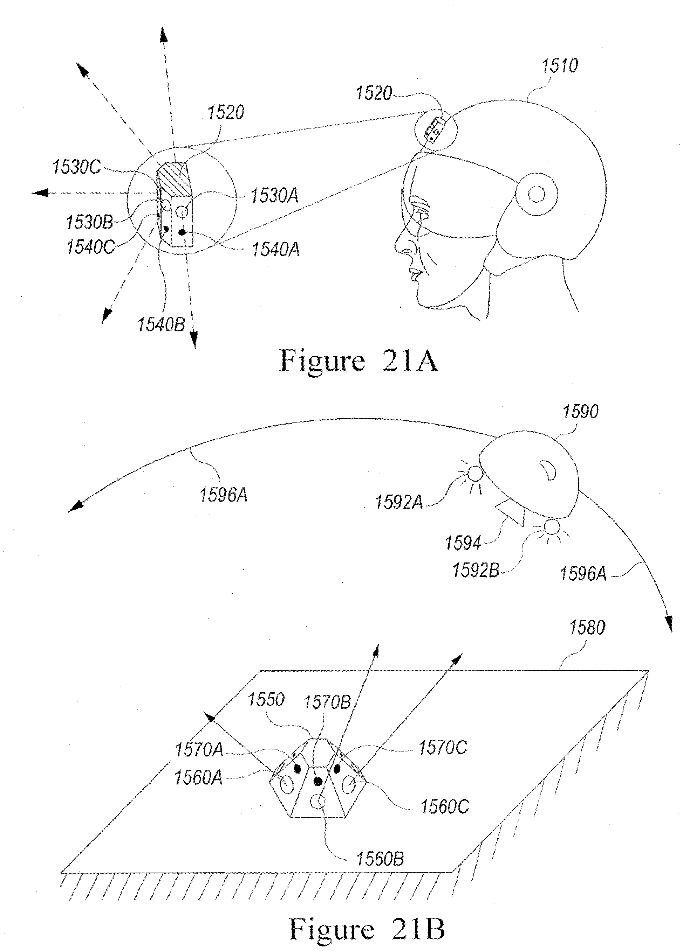

Figure 21A shows yet another embodiment according to the disclosed technique. In Figure 21A, an optical tracker sensor 1520 which comprises pixel array sensors 1530A-1530C and their corresponding LEDs 1540A-1540C is attached to a pilot helmet 1510, where the pilot is sitting in a cockpit facing an optical tracker sensor coupled with at least two visual indicators and affixed to the cockpit as a frame of reference (not shown). Advantageously, the helmet user is not limited ergonomically in its head orientation for a good operation of the tracker. This is achieved due to the wide field of view of the on-helmet optical tracker sensor 1520.

Figure 21B illustrates a stationary optical tracker unit 1550 comprising pixel array sensors 1560A-1560C and their corresponding LEDs 1570A-1570C, which are located along different surfaces of optical tracker unit 1550, each with a different radial angle with an additional tilt. A second optical tracking unit 1590 which includes two LEDS 1562A and 1592B and an optical tracker sensor 1494 are provided with better freedom of orientation and position (illustrated by arrows 1596A and 1596B) in the scene without losing tracking capabilities, due to the wider field of view of stationary tracking unit 1550. This configuration can be advantageous for instance when the stationary unit is attached to a patient and the moving unit is attached to a head mounted display system donned by a physician. In this situation the optical tracker keeps tracking the relative position and orientation between the head mounted system and the patient while the physician walks around the patient.

Aspects of the disclosed technique may take the form of an entirely hardware embodiment, an entirely software embodiment (including firmware, resident software, micro-code, etc.) or an embodiment combining software and hardware aspects.

In the above description, an embodiment is an example or implementation of the disclosed technique. The various appearances of "one embodiment," "an embodiment" or "some embodiments" do not necessarily all refer to the same embodiments.

Although various features of the disclosed technique may be described in the context of a single embodiment, the features may also be provided separately or in any suitable combination. Conversely, although the disclosed technique may be described herein in the context of separate embodiments for clarity, the disclosed technique may also be implemented in a single embodiment.

While the invention has been described with respect to a limited number of embodiments, these should not be construed as limitations on the scope of the invention, but rather as exemplifications of some of the preferred embodiments.

It will be appreciated by persons skilled in the art that the invention is not limited to what has been particularly shown and described hereinabove. Rather the scope of the invention is defined only by the claims, which follow.

## Claims

1. A medical Wide Field Of View optical tracking system for determining the position and orientation of at least one target object in a reference coordinate system comprising:

   at least one light emitter (356, 406, 462, 458, 506, $554_1$, $554_2$, $554_3$) attached to each of said at least one target object, said target object being at least one of a patient (322, 372, 422, 476, 520, 564) and a medical tool (418, 472, 514, 516, 566);
   at least two light emitters ($358_1$, $358_2$, $408_1$, $408_2$, $456_1$, $456_2$,) attached to a display (314, 364, 414, 468);
   at least two Wide Field Of View optical detectors (352 402, 454, 502, 552) attached to said at least one target object, a selected one being operative to be an active Wide Field Of View optical detector, said active Wide Field Of View optical detector acquiring at least one image of said at least two light emitters ($358_1$, $358_2$, $408_1$, $408_2$, $456_1$, $456_2$,) attached to said display (314, 364, 414, 468) when said at least two light emitters ($358_1$, $358_2$, $408_1$, $408_2$, $456_1$, $456_2$,) attached to said display are within the field of view thereof, each of said Wide Field Of View optical detector including:

      an optical sensor (102), for sensing light received from at least one light emitter within the field of view of said Wide Field Of View optical detector; and
      at least two optical receptors (104, 106), optically coupled with said optical sensor, each of said optical receptors including an entrance pupil (112, 114), said optical receptors (104, 106) being spatially spaced apart from each other, each of said optical receptors (104, 106) projecting a different angular section of an observed scene on said optical sensor;

   another optical detector (302, 354, 404, 452) attached to said display (314, 364, 414, 468), said another optical detector (302, 354, 404, 452) acquiring at least one image of said at least one light emitter (356, 406, 462, 458, 506, $554_1$, $554_2$, $554_3$) attached to said target object, within the field of view of said another optical detector (302, 354, 404, 452); and
   a position and orientation processor (310, 360, 410, 464, 510, 556), wirelessly coupled with said at least two Wide Field of View optical detectors (352, 402, 454, 502, 552) and coupled with said another optical detector (302, 354, 404, 452), said processor determining the position and orientation of each of said at least one target object in said reference coordinate system, according to representations of said at least one light emitter (356, 406, 462, 458, 506, $554_1$, $554_2$, $554_3$) attached to said target object and representations of said at least two other light emitters ($358_1$, $358_2$, $408_1$, $408_2$, $456_1$, $456_2$,) attached to said display (314, 364, 414, 468), said processor (310, 360, 410, 464, 510, 556) further rendering at least one of navigational information, a real-time image, a model of a region of interest of said patient (322, 372, 422, 476, 520, 564) and a representation associated with said medical tool (418, 472, 514, 516, 566), at least according to said position and orientation; and
   said display (314, 364, 414, 468), coupled with said position and orientation processor (310, 360, 410, 464, 510, 556), for displaying to a physician (318, 368, 420, 474, 522, 568) a rendered at least one of said navigational information, a real-time image, a model of region of interest of said patient and a representation associated with said medical tool (418, 472, 514, 516, 566), at a position and orientation corresponding to said determined position and orientation of said at least one target object.

2. The medical Wide Field Of View optical tracking system according to claim 1, wherein said navigational information, said real-time image, said model of a region of interest of said patient and said representation associated with said medical tool, each being associated with said reference coordinate system.

3. The medical Wide Field Of View optical tracking system according to claim 1, wherein said at least one of said navigational information, said real-time image, said model of a region of interest of said patient and said representation associated with said medical tool, is registered with one of the respective coordinate systems of at least another one of said navigational information, said real-time image, said model of a region of interest of said patient, said representation associated with said medical tool and with said reference coordinate system.

4. The medical Wide Field Of View optical tracking system according to claim 1, further including a database for storing said model, said navigational information and said representation associated with said medical tool.

5. The medical Wide Field Of View optical tracking system according to claim 1, wherein said display is a head mounted display configured to be donned by said physician.

6. The medical Wide Field Of View optical tracking system according to claim 1, wherein the angle between the optical axis of said display and said another optical detector attached to said display is fixed, and
wherein said processor adjusts said rendering of the displayed ones of said model, said navigational information, said representation associated with said medical tool and said real-time image to account for said angle.

7. The medical Wide Field Of View optical tracking system according to claim 1, wherein said target object includes at least said medical tool, said medical tool being a real-time imager, said real-time imager acquires said real-time image, and
wherein said real-time imager being one of:

   an ultrasound imager;
   a laparoscopic camera;
   an endoscope;
   a 2D X-ray imager;
   a 3D X-ray imager; and
   an MRI imager.

8. The medical Wide Field Of View optical tracking system according to claim 1, wherein said another optical detector is a Wide Field Of View optical detector including:

   an optical sensor, for sensing light received from said at least one light emitter; and
   at least two optical receptors, optically coupled with said optical sensor, each of said optical receptors including an entrance pupil, said optical receptors being spatially space apart from each other, each of said optical receptors projecting a different angular section of an observed scene on said optical sensor.

9. The medical Wide Field Of View optical tracking system according to claim 1, wherein said at least one light emitter attached to each of said at least one target object and said at least two light emitters attached to said display are light sources.

10. The medical Wide Field Of View optical tracking system according to claim 1, wherein said at least one light emitter attached to each of said at least one target object and said at least two light emitters attached to said display are light reflectors.

11. The medical Wide Field Of View optical tracking system according to claim 1, wherein said at least one light emitter attached to each of said at least one target object is a light source and said at least two light emitters attached to said display are light reflectors.

12. The medical Wide Field Of View optical tracking system according to claim 1, wherein said at least one light emitter attached to each of said at least one target object is a light reflector and said at least two light emitters attached to said display are light sources.

13. The medical Wide Field Of View optical tracking system according to claim 1, wherein at least two light emitters are attached to each of said at least one target object, each associated with a respective Wide Field Of View optical detector,
wherein only the light emitter associated with said active Wide Field Of View optical detector is employed for determining the position and orientation of said at least one target object.

14. The medical Wide Field Of View optical tracking system according to claim 13, wherein only the light emitter associated with said active wide field of view detector is activated.

**15.** The medical Wide Field Of View optical tracking system according to claim 1, wherein said processor further associates each representation of said at least two light emitters attached to said display within said field of view of said active Wide Field of View optical detector in said at least one image, with a respective one optical receptor projecting the light received from said at least two light emitters on said optical sensor.

wherein said position and orientation processor associates said at least one representation with a respective one optical receptor by at least one of tracking said representations,

determining a figure of merit for each association between said at least one representation and each optical receptor and selecting the association with the higher figure of merit,

according to the geometric configuration of said at least two optical receptors, and

based on a position and orientation provided by a low-accuracy tracking system.

**16.** The medical Wide Field Of View optical tracking system according to claim 1 wherein only at least one predefined region of interest within the images is processed by at least one of said at least one optical detector and said processor, wherein the borders of said region of interest are determined according to the predicted location of representations of the light emitters in said images, wherein said predicted location of said representations is determined according to one of on a low accuracy tracker,

the location of said representations in a previous at least one image, and

a prediction of the motion of said target object.

**17.** The medical Wide Field Of View optical tracking system according to claim 1, wherein said active Wide Field Of View optical detector is selected by tracking the location of said representations of said at least two light emitters attached to said display, in the image acquired by the optical sensor.

**Patentansprüche**

**1.** Medizinisches optisches Verfolgungssystem mit weitem Sichtfeld zum Bestimmen der Position und Ausrichtung mindestens eines Zielobjekts in einem Referenzkoordinatensystem, das Folgendes aufweist:

mindestens einen Lichtemitter (356, 406, 462, 458, 506, $554_1$, $554_2$, $554_3$), der an jedem des mindestens einen Zielobjekts angebracht ist, wobei das Zielobjekt ein Patient (322, 372, 422, 476, 520, 564) und/oder ein medizinisches Gerät (418, 472, 514, 516, 566) ist;

mindestens zwei Lichtemitter ($358_1$, $358_2$, $408_1$, $408_2$, $456_1$, $456_2$), die an einer Anzeige (314, 364, 414, 468) angebracht sind;

mindestens zwei optische Detektoren (352 402, 454, 502, 552) mit weitem Sichtfeld, die an dem mindestens einen Zielobjekt angebracht sind, wobei ein ausgewählter davon als ein aktiver optischer Detektor mit weitem Sichtfeld betreibbar ist, wobei der aktive optische Detektor mit weitem Sichtfeld mindestens ein Bild der mindestens zwei Lichtemitter ($358_1$, $358_2$, $408_1$, $408_2$, $456_1$, $456_2$), die an der Anzeige (314, 364, 414, 468) angebracht sind, erfasst, wenn sich die mindestens zwei Lichtemitter ($358_1$, $358_2$, $408_1$, $408_2$, $456_1$, $456_2$), die an der Anzeige angebracht sind, in dem Sichtfeld davon befinden, wobei jeder der optischen Detektoren mit weitem Sichtfeld Folgendes aufweist:

einen optischen Sensor (102) zum Abtasten von Licht, das von mindesten einem Emitter innerhalb des Sichtfelds des optischen Detektors mit weitem Sichtfeld empfangen wird; und

mindestens zwei optische Rezeptoren (104, 106), die optisch mit dem optischen Sensor gekoppelt sind, wobei jeder der optischen Rezeptoren eine Eintrittspupille (112, 114) aufweist, wobei die optischen Rezeptoren (104, 106) voneinander beabstandet sind, wobei jeder der optischen Rezeptoren (104, 106) einen unterschiedlichen Winkelbereich einer beobachteten Szene auf dem optischen Sensor projiziert;

einen weiteren optischen Detektor (302, 354, 404, 452), der an der Anzeige (314, 364, 414, 468) angebracht ist, wobei der weitere optische Detektor (302, 354, 404, 452) mindestens ein Bild des mindestens einen Lichtemitters (356, 406, 462, 458, 506, $554_1$, $554_2$, $554_3$), der an dem Zielobjekt angebracht ist, innerhalb des Sichtfelds des weiteren optischen Detektors (302, 354, 404, 452) erfasst; und

einen Positions- und Ausrichtungsprozessor (310, 360, 410, 464, 510, 556), der drahtlos mit den mindestens zwei optischen Detektoren (352, 402, 454, 502, 552) mit weitem Sichtfeld gekoppelt ist und mit dem weiteren optischen Detektor (302, 354, 404, 452) gekoppelt ist, wobei der Prozessor die Position und Ausrichtung jedes des mindestens einen Zielobjekts in dem Referenzkoordinatensystem gemäß Darstellungen des mindestens einen Lichtemitters (356, 406, 462, 458, 506, $554_1$, $554_2$, $554_3$), der an dem Zielobjekt angebracht ist, und Darstellungen der mindestens zwei weiteren Lichtemitter ($358_1$, $358_2$, $408_1$, $408_2$, $456_1$,

$456_2$), die an der Anzeige (314, 364, 414, 468) angebracht sind, bestimmt, wobei der Prozessor (310, 360, 410, 464, 510, 556) ferner Navigationsinformationen und/oder ein Echtzeitbild und/oder ein Modell einer Region von Interesse des Patienten (322, 372, 422, 476, 520, 564) und/oder eine Darstellung, die dem medizinischen Gerät (418, 472, 514, 516, 566) zugeordnet ist, zumindest gemäß der Position und Ausrichtung, wiedergibt; und

die Anzeige (314, 364, 414, 468), die mit dem Positions- und Ausrichtungsprozessor (310, 360, 410, 464, 510, 556) gekoppelt ist, zum Anzeigen wiedergegebener Navigationsinformationen und/oder eines Echtzeitbilds und/oder eines Modells einer Region von Interesse des Patienten und/oder einer Darstellung, die dem medizinischen Gerät (418, 472, 514, 516, 566) zugeordnet ist, an einer Position und Ausrichtung, die der bestimmten Position und Ausrichtung des mindestens einen Zielobjekts entsprechen, für einen Arzt (318, 368, 420, 474, 522, 568).

2. Medizinisches optisches Verfolgungssystem mit weitem Sichtfeld nach Anspruch 1, wobei die Navigationsinformationen, das Echtzeitbild, das Modell einer Region von Interesse des Patienten und die Darstellung, die dem medizinischen Gerät zugeordnet ist, jeweils dem Referenzkoordinatensystem zugeordnet sind.

3. Medizinisches optisches Verfolgungssystem mit weitem Sichtfeld nach Anspruch 1, wobei die Navigationsinformationen und/oder das Echtzeitbild, und/oder das Modell einer Region von Interesse des Patienten und/oder die Darstellung, die dem medizinischen Gerät zugeordnet ist, mit einem der jeweiligen Koordinatensysteme mindestens eines anderen der Navigationsinformationen, des Echtzeitbilds, des Modells einer Region von Interesse des Patienten, der Darstellung, die einem medizinischen Gerät zugeordnet ist, und/oder mit dem Referenzkoordinatensystem verzeichnet.

4. Medizinisches optisches Verfolgungssystem mit weitem Sichtfeld nach Anspruch 1, das ferner eine Datenbank zum Speichern des Modells, der Navigationsinformationen und der Darstellung, die dem medizinischen Gerät zugeordnet ist, aufweist.

5. Medizinisches optisches Verfolgungssystem mit weitem Sichtfeld nach Anspruch 1, wobei die Anzeige eine am Kopf befestigte Anzeige ist, die dazu ausgelegt ist, von dem Arzt getragen zu werden.

6. Medizinisches optisches Verfolgungssystem mit weitem Sichtfeld nach Anspruch 1, wobei der Winkel zwischen der optischen Achse der Anzeige und dem weiteren optischen Detektor, der an der Anzeige angebracht ist, stabil ist, und wobei der Prozessor die Wiedergabe des Angezeigten von Modell, Navigationsinformationen, Darstellung, die dem medizinischen Gerät zugeordnet ist, und Echtzeitbild anpasst, um den Winkel zu berücksichtigen.

7. Medizinisches optisches Verfolgungssystem mit weitem Sichtwinkel nach Anspruch 1, wobei das Zielobjekt mindestens das medizinische Gerät aufweist, wobei das medizinische Gerät ein Echtzeit-Imager ist, wobei der Echtzeit-Imager das Echtzeitbild erfasst, und wobei der Echtzeit-Imager einer der folgenden ist:

ein Ultraschall-Imager;
eine laparoskopische Kamera;
ein Endoskop;
ein 2D-Röntgen-Imager;
ein 3D-Röntgen-Imager; und
ein MRI-Imager.

8. Medizinisches optisches Verfolgungssystem mit weitem Sichtfeld nach Anspruch 1, wobei der weitere optische Detektor ein optischer Detektor mit weitem Sichtfeld ist, der Folgendes aufweist:

einen optischen Sensor zum Abtasten von Licht, das von mindestens einem Lichtemitter empfangen wird; und mindestens zwei optische Rezeptoren, die optisch mit dem optischen Sensor gekoppelt sind, wobei jeder der optischen Rezeptoren eine Eintrittspupille aufweist, wobei die optischen Rezeptoren voneinander beabstandet sind, wobei jeder der optischen Rezeptoren einen unterschiedlichen Winkelbereich einer beobachteten Szene auf dem optischen Sensor projiziert.

9. Medizinisches optisches Verfolgungssystem mit weitem Sichtfeld nach Anspruch 1, wobei der mindestens eine Lichtemitter, der an jedem des mindestens einen Zielobjekts angebracht ist, und die mindestens zwei Lichtemitter, die an der Anzeige angebracht sind, Lichtquellen sind.

10. Medizinisches optisches Verfolgungssystem mit weitem Sichtfeld nach Anspruch 1, wobei der mindestens eine Lichtemitter, der an jedem des mindestens einen Zielobjekts angebracht ist, und die mindestens zwei Lichtemitter, die an der Anzeige angebracht sind, Lichtreflektoren sind.

11. Medizinisches optisches Verfolgungssystem mit weitem Sichtfeld nach Anspruch 1, wobei der mindestens eine Lichtemitter, der an jedem des mindestens einen Zielobjekts angebracht ist, eine Lichtquelle ist und die mindestens zwei Lichtemitter, die an der Anzeige angebracht sind, Lichtreflektoren sind.

12. Medizinisches optisches Verfolgungssystem mit weitem Sichtfeld nach Anspruch 1, wobei der mindestens eine Lichtemitter, der an jedem des mindestens einen Zielobjekts angebracht ist, ein Lichtreflektor ist und die mindestens zwei Lichtemitter, die an der Anzeige angebracht sind, Lichtquellen sind.

13. Medizinisches optisches Verfolgungssystem mit weitem Sichtfeld nach Anspruch 1, wobei mindestens zwei Lichtemitter an jedem des mindestens einen Zielobjekts angebracht sind, wobei jeder einem entsprechenden optischen Detektor mit weitem Sichtfeld zugeordnet ist,
wobei nur der Lichtemitter, der dem aktiven optischen Detektor zugeordnet ist, zum Bestimmen der Position und Ausrichtung des mindestens einen Zielobjekts verwendet wird.

14. Medizinisches optisches Verfolgungssystem mit weitem Sichtwinkel nach Anspruch 13, wobei nur der Lichtemitter, der dem aktiven Detektor mit weitem Sichtfeld zugeordnet ist, aktiviert ist.

15. Medizinisches optisches Verfolgungssystem mit weitem Sichtwinkel nach Anspruch 1, wobei der Prozessor ferner jede Darstellung der mindestens zwei Lichtemitter, die an der Anzeige angebracht sind, innerhalb des Sichtfelds des aktiven optischen Detektors mit weitem Sichtfeld in dem mindestens einen Bild einem jeweiligen optischen Rezeptor, der das Licht, das von den mindestens zwei Lichtemittern auf dem optischen Sensor empfangen wird, zuordnet,
wobei der Positions- und Ausrichtungsprozessor die mindestens eine Darstellung einem jeweiligen optischen Rezeptor zuordnet, mittels Verfolgens der Darstellungen und/oder
Bestimmen einer Gütezahl für jede Zuordnung zwischen der mindestens einen Darstellung und jedes optischen Rezeptors und/oder Auswählen der Zuordnung mit der höchsten Gütezahl,
gemäß dem geometrischen Aufbau der mindestens zwei optischen Rezeptoren, und
basierend auf einer Position und Ausrichtung, die von einem Verfolgungssystem mit geringer Genauigkeit bereitgestellt werden.

16. Medizinisches optisches Verfolgungssystem mit weitem Sichtfeld nach Anspruch 1, wobei nur mindestens eine vorbestimmte Region von Interesse innerhalb des Bilds von mindestens einem des optischen Detektors und/oder des Prozessors verarbeitet wird,
wobei die Grenzen der Region von Interesse gemäß der vorausgesagten Stelle der Darstellungen der Lichtemitter in dem Bild bestimmt werden, wobei die vorausgesagten Stellen der Darstellungen gemäß einem von auf einem Tracker mit geringer Genauigkeit bestimmt werden,
die Stelle der Darstellungen in einem vorherigen mindestens einem Bild, und
eine Vorhersage der Bewegung des Zielobjekts.

17. Medizinisches optisches Verfolgungssystem mit weitem Sichtfeld nach Anspruch 1, wobei der aktive optische Detektor mit weitem Sichtfeld durch Verfolgen der Stelle der Darstellungen der mindestens zwei Lichtemitter, die an der Anzeige angebracht sind, in dem Bild, das von dem optischen Sensor erfasst wird, ausgewählt wird.

**Revendications**

1. Système de suivi optique à large champ de vision pour la détermination de la position et de l'orientation d'au moins un objet cible dans un système de coordonnées de référence, comprenant :

au moins un émetteur de lumière ($356, 406, 462, 458, 506, 554_1, 554_2, 554_3$) rattaché à chacun dudit au moins un objet cible, ledit objet cible étant au moins un objet parmi un patient ($322, 372, 422, 476, 520, 564$) et un outil médical ($418, 472, 514, 516, 566$) ;

au moins deux émetteurs de lumière ($358_1$, $358_2$, $408_1$, $408_2$, $456_1$, $456_2$) rattachés à un afficheur (314, 364, 414, 468) ;

au moins deux détecteurs optiques à large champ de vision (352, 402, 454, 502, 552) rattachés audit au moins un objet cible, un détecteur sélectionné étant opérationnel en tant que détecteur optique actif à large champ de vision, ledit détecteur optique actif à large champ de vision acquérant au moins une image desdits au moins deux émetteurs de lumière ($358_1$, $358_2$, $408_1$, $408_2$, $456_1$, $456_2$) rattachés audit afficheur (314, 364, 414, 468) lorsque lesdits au moins deux émetteurs de lumière ($358_1$, $358_2$, $408_1$, $408_2$, $456_1$, $456_2$) rattachés audit afficheur se trouvent dans son champ de vision, chacun desdits détecteurs optiques à large champ de vision incluant : un capteur optique (102) pour détecter la lumière reçue en provenance d'au moins un émetteur de lumière dans le champ de vision dudit détecteur optique à large champ de vision ; et

au moins deux récepteurs optiques (104, 106) couplés au niveau optique audit capteur optique, chacun desdits récepteurs optiques incluant une pupille d'entrée (112, 114), lesdits récepteurs optiques (104, 106) étant espacés spatialement les uns des autres, chacun desdits récepteurs optiques (104, 106) projetant une section angulaire différente d'une scène observée sur ledit capteur optique ;

un autre détecteur optique (302, 354, 404, 452) rattaché audit afficheur (314, 364, 414, 468), ledit autre détecteur optique (302, 354, 404, 452) acquérant au moins une image dudit au moins un émetteur de lumière (356, 406, 462, 458, 506, $554_1$, $554_2$, $554_3$) rattaché audit objet cible dans le champ de vision dudit autre détecteur optique (302, 354, 404, 452) ; et

un processeur de position et d'orientation (310, 360, 410, 464, 510, 556) couplé sans fil auxdits au moins deux détecteurs optiques à large champ de vision (352, 402, 454, 502, 552) et couplé à l'autre détecteur optique (302, 354, 404, 452), ledit processeur déterminant la position et l'orientation de chacun dudit au moins un objet cible dans ledit système de coordonnées de référence en fonction de représentations dudit au moins un émetteur de lumière (356, 406, 462, 458, 506, $554_1$, $554_2$, $554_3$) rattaché audit objet cible et de représentations desdits au moins deux autres émetteurs de lumière ($358_1$, $358_2$, $408_1$, $408_2$, $456_1$, $456_2$) rattachés audit afficheur (314, 364, 414, 468), ledit processeur (310, 360, 410, 464, 510, 556) restituant en outre au moins un élément parmi une information de navigation, une image en temps réel, un modèle d'une zone d'intérêt dudit patient (322, 372, 422, 476, 520, 564) et une représentation associée au dit outil médical (418, 472, 514, 516, 566) au moins en fonction desdites position et orientation ; et

ledit afficheur (314, 364, 414, 468) couplé audit processeur de position et d'orientation (310, 360, 410, 464, 510, 556) pour afficher à un médecin (318, 368, 420, 474, 522, 568) au moins un élément restitué parmi ladite information de navigation, une image en temps réel, un modèle d'une zone d'intérêt dudit patient et une représentation associée audit outil médical (418, 472, 514, 516, 566) à une position et une orientation correspondant auxdites position et orientation déterminées dudit au moins un objet cible.

2.  Système de suivi optique à large champ de vision selon la revendication 1, dans lequel ladite information de navigation, ladite image en temps réel, ledit modèle d'une zone d'intérêt dudit patient et ladite représentation associée audit outil médical sont chacun associés audit système de coordonnées de référence.

3.  Système de suivi optique à large champ de vision selon la revendication 1, dans lequel au moins un élément parmi ladite information de navigation, ladite image en temps réel, ledit modèle d'une zone d'intérêt dudit patient et ladite représentation associée audit outil médical est enregistré avec un des systèmes de coordonnées respectifs d'au moins un autre élément parmi ladite information de navigation, ladite image en temps réel, ledit modèle d'une zone d'intérêt dudit patient, ladite représentation associée audit outil médical et avec ledit système de coordonnées de référence.

4.  Système de suivi optique à large champ de vision selon la revendication 1, comprenant en outre une base de données pour sauvegarder ledit modèle, ladite information de navigation et ladite représentation associée au dit outil médical.

5.  Système de suivi optique à large champ de vision selon la revendication 1, dans lequel ledit afficheur est un afficheur se montant sur la tête et conçu pour être porté par ledit médecin.

6.  Système de suivi optique à large champ de vision selon la revendication 1, dans lequel l'angle entre l'axe optique dudit afficheur et ledit autre détecteur optique rattaché audit afficheur est fixe, et

ledit processeur règle ladite restitution des éléments affichés parmi ledit modèle, ladite information de navigation, ladite représentation associée audit outil médical et ladite image en temps réel pour prendre en compte ledit angle.

7.  Système de suivi optique à large champ de vision selon la revendication 1, dans lequel ledit objet cible inclut au moins ledit outil médical, ledit outil médical étant un imageur en temps réel, ledit imageur en temps réel acquérant ladite image en temps réel, et

ledit imageur en temps réel étant un appareil parmi :

un imageur à ultrasons ;
une caméra laparoscopique ;
un endoscope ;
un imageur radiographique en 2D ;
un imageur radiographique en 3D ; et
un imageur IRM.

8. Système de suivi optique à large champ de vision selon la revendication 1, dans lequel ledit autre détecteur optique est un détecteur optique à large champ de vision incluant :

un capteur optique pour la détection de la lumière reçue en provenance dudit au moins un émetteur de lumière ; au moins deux récepteurs optiques couplés au niveau optique audit capteur optique, chacun desdits récepteurs optiques incluant une pupille d'entrée, lesdits récepteurs optiques étant espacés spatialement les uns des autres, chacun desdits récepteurs optiques projetant une section angulaire différente d'une scène observée sur ledit capteur optique.

9. Système de suivi optique à large champ de vision selon la revendication 1, dans lequel ledit au moins un émetteur de lumière rattaché à chacun dudit au moins un objet cible et lesdits au moins deux émetteurs de lumière rattachés audit afficheur sont des sources de lumière.

10. Système de suivi optique à large champ de vision selon la revendication 1, dans lequel ledit au moins un émetteur de lumière rattaché à chacun dudit au moins un objet cible et lesdits au moins deux émetteurs de lumière rattachés audit afficheur sont des réflecteurs de lumière.

11. Système de suivi optique à large champ de vision selon la revendication 1, dans lequel ledit au moins un émetteur de lumière rattaché à chacun dudit au moins un objet cible est une source de lumière et lesdits au moins deux émetteurs de lumière rattachés audit afficheur sont des réflecteurs de lumière.

12. Système de suivi optique à large champ de vision selon la revendication 1, dans lequel dans lequel ledit au moins un émetteur de lumière rattaché à chacun dudit au moins un objet cible est un réflecteur de lumière et lesdits au moins deux émetteurs de lumière rattachés audit afficheur sont des sources de lumière.

13. Système de suivi optique à large champ de vision selon la revendication 1, dans lequel au moins deux émetteurs de lumière sont rattachés à chacun dudit au moins un objet cible, chacun étant associé à un détecteur optique respectif à large champ de vision,
seul l'émetteur de lumière associé audit détecteur actif à large champ de vision est employé pour déterminer la position et l'orientation dudit au moins un objet cible.

14. Système de suivi optique à large champ de vision selon la revendication 13, dans lequel seul l'émetteur de lumière associé audit détecteur actif à large champ de vision est activé.

15. Système de suivi optique à large champ de vision selon la revendication 1, dans lequel ledit processeur associe en outre chaque représentation desdits au moins deux émetteurs de lumière rattachés au dit afficheur dans ledit champ de vision dudit détecteur optique actif à large champ de vision dans ladite au moins une image, un récepteur optique respectif projetant la lumière reçue en provenance desdits au moins deux émetteurs de lumière sur ledit capteur optique,
ledit processeur de position et d'orientation associe ladite au moins une représentation à un récepteur optique respectif par au moins une action parmi le suivi desdites représentations,
la détermination d'un facteur de mérite pour chaque association entre ladite au moins une représentation et chaque récepteur optique et la sélection de l'association ayant le facteur de mérite le plus élevé,
en fonction de la configuration géométrique desdits au moins deux récepteurs optiques, et
en se basant sur une position et une orientation fournie par un système de suivi à faible précision.

16. Système de suivi optique à large champ de vision selon la revendication 1, dans lequel seule au moins une zone prédéfinie d'intérêt dans les images est traitée par au moins un parmi ledit au moins un détecteur optique et ledit processeur,

les limites de ladite zone d'intérêt sont déterminées en fonction de l'emplacement prévisionnel de représentation des émetteurs de lumière dans lesdites images, ledit emplacement prévisionnel desdites représentations étant déterminé en fonction d'un élément parmi un suiveur de faible précision,

l'emplacement desdites représentations dans une précédent au moins une image, et

une prévision du mouvement dudit objet cible.

17. Système de suivi optique à large champ de vision selon la revendication 1, dans lequel ledit détecteur optique actif à large champ de vision est sélectionné en suivant l'emplacement desdites représentations desdits au moins deux émetteurs de lumière rattachés audit afficheur dans l'image acquise par le capteur optique.

FIG. 1
PRIOR ART

FIG. 2A

FIG. 2B

FIG. 3A

FIG. 3B

EP 3 076 892 B1

EP 3 076 892 B1

```
                            ┌─ 200

        MOVING OBJECT                    REFERENCE LOCATION

      ┌─────────────────┐              ┌─────────────────┐
      │  MOVING LIGHT   │              │ REFERENCE LIGHT │
      │    EMITTER      │              │    EMITTER      │
      └─────────────────┘              └─────────────────┘
              └─ 212                      204₁ ┘

      ┌─────────────────┐              ┌─────────────────┐
      │ MOVING OPTICAL  │              │   REFERENCE     │
      │    DETECTOR     │              │ OPTICAL DETECTOR│
      └─────────────────┘              └─────────────────┘
208            └─ 210                     206 ┘           202

                                       ┌─────────────────┐
                                       │ REFERENCE LIGHT │
                                       │    EMITTER      │
                                       └─────────────────┘
                                          204₂ ┘

              ┌─────────────────┐
              │     POSE        │
              │   PROCESSOR     │
              └─────────────────┘
                    └─ 214
```

FIG. 4

FIG. 5C

FIG. 5D

FIG. 5A

FIG. 5B

FIG. 6

MOVING OBJECT

MOVING LIGHT
EMITTER

260

MOVING OPTICAL
DETECTOR

258

252

REFLECTIVE SURFACE

254

ORIENTATION
PROCESSOR

256

250

**FIG. 7**

REFLECTIVE SURFACE 254

262

β

252

264

FIG. 8

FIG. 9

EP 3 076 892 B1

**FIG. 10**

**FIG. 11**

FIG. 12

**FIG. 13**

EP 3 076 892 B1

FIG. 14

**FIG. 15**

FIG. 16A

FIG. 16B

FIG. 16C

FIG. 16D

FIG. 16E

Figure 17

Figure 18

51

Figure 19

Figure 20

EP 3 076 892 B1

Figure 21A

Figure 21B

54

**REFERENCES CITED IN THE DESCRIPTION**

**Patent documents cited in the description**

- US 3678283 A, LaBaw
- US 5767524 A, Barbier
- WO 2009040792 A, Yahav
- US 20040138665 A, Cosman
- US 20110079703 A, Gunning